(19) Europäisches Patentamt

European Patent Office

Office européen des brevets

(11) **EP 4 424 418 A1**

(12) **EUROPEAN PATENT APPLICATION**

published in accordance with Art. 153(4) EPC

(43) Date of publication:
**04.09.2024 Bulletin 2024/36**

(21) Application number: **22885918.7**

(22) Date of filing: **25.10.2022**

(51) International Patent Classification (IPC):
**B01J 29/068** (2006.01)   **C07C 13/28** (2006.01)

(52) Cooperative Patent Classification (CPC):
**B01J 29/068; C07C 13/28;** Y02P 20/52

(86) International application number:
**PCT/CN2022/127278**

(87) International publication number:
**WO 2023/072041 (04.05.2023 Gazette 2023/18)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 26.10.2021  CN 202111250651
28.06.2022  CN 202210754110
28.06.2022  CN 202210754120

(71) Applicants:
• **China Petroleum & Chemical Corporation
Beijing 100728 (CN)**
• **Shanghai Research Institute of Petrochemical
Technology, SINOPEC
Shanghai 201208 (CN)**

(72) Inventors:
• **LAN, Dawei
Shanghai 201208 (CN)**
• **YANG, Weimin
Shanghai 201208 (CN)**
• **WANG, Zhendong
Shanghai 201208 (CN)**
• **LI, Xiangcheng
Shanghai 201208 (CN)**
• **LIU, Chuang
Shanghai 201208 (CN)**
• **LI, Junjie
Shanghai 201208 (CN)**
• **WANG, Wennian
Shanghai 201208 (CN)**

(74) Representative: **Hoffmann Eitle
Patent- und Rechtsanwälte PartmbB
Arabellastraße 30
81925 München (DE)**

(54) **HYDROGENATION-ACID CATALYSIS BIFUNCTIONAL CATALYST, AND PREPARATION METHOD THEREFOR AND APPLICATION THEREOF**

(57)     Disclosed is a hydrogenation-acid catalysis bifunctional catalyst, based on the mass of the catalyst, containing 80-99.8% of a silica-alumina molecular sieve component, 0.2-2% of a metal component with hydrogenation activity supported on the molecular sieve, and 0-20% of a hydrocarbyl modifying component, wherein the hydrogenation active metal is selected from ruthenium, platinum, palladium, copper, nickel, or a combination thereof, the hydrocarbyl modifying component is a $C_{1-20}$ hydrocarbyl. The catalyst has dual functions of hydrogenation and acid catalysis, and is suitable for benzene hydroalkylation reaction and alkane hydroisomerization reaction. Moreover, when used in the benzene hydroalkylation to produce cyclohexylbenzene, the catalyst has the characteristics of high benzene conversion rate, good product selectivity, and less by-product cyclohexane.

Fig. 1

EP 4 424 418 A1

## Description

### Technical field

The present application relates to the field of catalyst, and specifically to a hydrogenation-acid catalysis bifunctional catalyst and preparation method and use thereof.

### Background Art

Cyclohexylbenzene is an important chemical product with important applications in the fields of liquid crystal and rechargeable battery. Among them, cyclohexylbenzene liquid crystal has extremely high chemical stability, photochemical stability and excellent physical properties, and is one of the ideal materials for liquid crystal displays. Cyclohexylbenzene can also be used as an additive component in the electrolyte of lithium-ion battery. It has the function of preventing overcharge and can effectively improve battery safety. In addition, important chemical products phenol and cyclohexanone can be obtained by using cyclohexylbenzene as an intermediate through further peroxidation and decomposition reactions, which can then be used in the production of phenolic resin, caprolactam and nylon. Therefore, the preparation and production of cyclohexylbenzene have received widespread attention. The basic information of cyclohexylbenzene is as follows: colorless liquid, CAS number: 827-52-1, the density of $0.95 g/cm^3$, the boiling point of 238-240 °C, the melting point of 5°C, and the flash point of 98 °C.

Depending on the raw materials, the main methods for preparing cyclohexylbenzene include: hexene alkylbenzene cyclization method, and benzene hydroalkylation method. Among them, the basic principle of the benzene hydroalkylation method is: benzene and hydrogen are used as raw materials, part of benzene is hydrogenated at the metal active center to obtain a 6-membered cyclic olefin structure (such as cyclohexene, etc.), which further undergoes an alkylation reaction with benzene at the position of acidic active center to obtain the cyclohexylbenzene product. Therefore, a bifunctional catalyst with both a hydrogenation center and an alkylation active center can be used in the production process of cyclohexylbenzene.

Research on the hydroalkylation of benzene to prepare cyclohexylbenzene first began in the 1980s. The catalysts currently developed mainly use metals supported on molecular sieves, and most of them suffer from slow catalytic efficiency and low selectivity. For example, catalysts based on MCM-22 series molecular sieves (US2011/0015457A1, CN104105679A) have the problems of slow catalytic rate and high selectivity for the by-product cyclohexane. Other catalysts such as those using Ni-rare earth treated HY molecular sieve as a carrier (US4219689) have the problems of low benzene conversion rate and low yield of the cyclohexylbenzene product. It was subsequently reported (Molecular Catalysis 2017, 442, 27-38) that Pd/HY supported molecular sieve was used as a catalyst to catalyze the hydroalkylation of benzene to prepare cyclohexylbenzene in one step. The initial conversion rate of benzene was 42.2%, and the selectivity of cyclohexylbenzene was maintained at about 75%, and the selectivity of the excessive hydrogenation by-product cyclohexane was as high as about 20%. From this point of view, the existing technology mainly has the problems of poor product selectivity and more cyclohexane as a by-product, which brings great problems to practical industrial application.

Similarly, other hydrogenation-solid acid bifunctional catalytic reactions also face the problems of numerous by-products and reduced selectivity of target products. For example, patent CN112934251A discloses a scheme of using hydrogenation metal-mordenite in n-heptane hydroisomerization reaction, and its isoheptane selectivity is about 60 %-70%. The earlier US5643440, US5302279, US6190532, etc. discloses a scheme of using precious metal-low acidity molecular sieve to catalyze the isomerization of heavy oil. However, there is an overall lack of positional control of the hydrogenation and acid catalytic center sites, and the optimal matching effect of the dual active centers is not shown.

### Disclosure of the Invention

The aim of the present application is to provide a hydrogenation-acid catalysis bifunctional catalyst and its preparation method and application. The catalyst has dual functions of hydrogenation and acid catalysis, and is suitable for benzene hydroalkylation reaction and alkane hydroisomerization reaction. Moreover, when used in the benzene hydroalkylation to produce cyclohexylbenzene, the catalyst has the characteristics of high benzene conversion rate, good product selectivity, and less by-product cyclohexane.

In order to achieve the above aim, on one hand, the present application provides a hydrogenation-acid catalysis bifunctional catalyst, based on the mass of the catalyst, containing 80-99.8% of a silica-alumina molecular sieve component, 0.2-2% of a metal component with hydrogenation activity supported on the molecular sieve, and 0-20% of a hydrocarbyl modifying component, wherein the hydrogenation active metal is selected from ruthenium, platinum, palladium, copper, nickel, or a combination thereof, the hydrocarbyl modifying component is a $C_{1-20}$ hydrocarbyl.

On the other hand, a method for preparing the catalyst of the present application is provided, comprising the following steps:

(1) providing a H-type silica-alumina molecular sieve; and
(2) supporting the hydrogenation active metal on the H-type silica-alumina molecular sieve, and optionally performing hydrocarbylation treatment and/or reduction on the resulting product to obtain the catalyst.

On the other hand, use of the catalyst of the present application in hydrocarbon hydroconversion reaction is provided, including the step of contacting and reacting the hydrocarbon feedstock with the catalyst in the presence of hydrogen.

On the other hand, the present application provides a one-step method for producing cyclohexylbenzene by hydrogenating benzene, including the step of contacting and reacting benzene with the catalyst of the present application in the presence of hydrogen to obtain cyclohexylbenzene.

On the other hand, the present application provides an alkane hydroisomerization method, including the step of contacting and reacting a linear alkane with the catalyst of the present application in the presence of hydrogen to obtain an isomerization product, wherein the linear alkane is a C8 or higher linear alkane.

Compared with the prior art, the beneficial effects of the catalyst of the present application include:

1. the catalyst of the present application has dual functions of hydrogenation and acid catalysis, and can realize the hydroalkylation reaction of benzene to generate cyclohexylbenzene under mild reaction conditions. The benzene conversion rate and the selectivity of the main product cyclohexylbenzene are both very high, and the reaction system has good stability; especially, when using silica-alumina molecular sieve with ATS structure as the molecular sieve component, the catalyst of the present application has a special pore structure and acidic characteristics, which has obvious effect on reducing cyclohexane and dicyclohexylbenzene by-products in benzene hydroalkylation reaction;

2. the catalyst of the present application has a specific composition, especially its active metal component is mainly concentrated in the molecular sieve channels, and the external surface has low metal content, thus ensuring that the content of excessive hydrogenation by-products (such as cyclohexane ) is less; and the external surface of the catalyst is highly hydrophobic, so it has better affinity with various types of alkanes, aromatics and other non-polar substances, ensuring that the benzene conversion rate remains at a high level ; and

3. when the catalyst of the present application is used for the isomerization of linear alkanes, since the metal is mainly dispersed in the molecular sieve channels and the spatial distance from the strong acid sites is small, and the amount of acid on the external surface is small, it has the advantages of high substrate conversion rate and good selectivity of hydroisomerization product in the alkane hydroisomerization reaction.

Other features and advantages of the present application will be described in detail in the following detailed description.

## Brief Description of the Drawings

The accompanying drawings are used to provide a further understanding of the present application and constitute a part of the specification. They are used to explain the present application together with the following specific embodiments, but do not constitute limitation of the present application. In the drawings:

Figure 1 is a XRD spectrum of the catalyst prepared in Preparation Example I-1;
Figure 2 is a TEM image of the catalyst prepared in Preparation Example I-1;
Figure 3 is an SEM image of the catalyst prepared in Preparation Example I-1;
Figure 4 is a XRD spectrum of the catalyst prepared in Preparation Example II-1;
Figure 5 is an infrared absorption spectrum of the catalyst prepared in Preparation Example II-1;
Figure 6 is a XRD spectrum of the catalyst prepared in Preparation Example II-3; and
Figure 7 is a XRD spectrum of the catalyst prepared in Preparation Example III-1.

## Detailed Description of the Invention

Specific embodiments of the present application will be described in detail below with reference to the accompanying drawings. It should be understood that the specific embodiments described here are only used to illustrate and explain the present application, and are not intended to limit the present application.

Any specific numerical value disclosed herein (including the endpoints of a numerical range) is not limited to the precise value of the numerical value, but is to be understood to also encompass values close to the precise value, such as all possible values within ±5% of the precise value. Moreover, for the disclosed numerical range, one or more new numerical ranges can be obtained by any combination between the endpoint values of the range, between the endpoint values and specific point values within the range, and between each specific point value, and these new numerical ranges should also be deemed to be specifically disclosed herein.

In the context of the specification, the so-called "silicon-aluminum ratio" or "silicon-aluminum molar ratio" refers to the molar ratio between silicon, calculated as $SiO_2$, and aluminum, calculated as $Al_2O_3$, in the molecular sieve.

In the context of the specification, in the XRD data of molecular sieves, w, m, s, vs, w-m, m-s and s-vs etc. represent the relative intensity $I/I_0$ of the diffraction peak at the corresponding 2θ angle relative to the strongest diffraction peak (i.e., the diffraction peak with the highest intensity) calculated based on the diffraction peak intensity (in terms of peak height), where I represents the peak intensity of the corresponding diffraction peak and $I_0$ represents the peak intensity of the strongest diffraction peak, w represents weak, m represents medium, s represents strong, vs represents very strong, w-m represents weak to medium, m-s represents medium to strong, and s-vs represents strong to very strong. This representation is well known to those skilled in the art. Generally speaking, w represents less than 20; m represents 20-40; s represents 40-70; vs represents greater than 70, w-m represents less than 40, m-s represents 20-70, and s-vs represents greater than 40.

According to the present application, the interplanar spacing of each diffraction peak in the XRD diffraction spectrum of the molecular sieve can be calculated based on the 2θ value of the diffraction peak through the Bragg formula: $\lambda=2d\sin\theta$ (where λ is the wavelength of the incident wave, λ=1.54Å, and d is the interplanar spacing, θ is the angle between the incident light and the scattering plane).

In the context of the specification, the so-called "pore volume" refers to the volume of pores per unit mass of the catalyst. The so-called "total pore volume" refers to the volume of all pores per unit mass of the catalyst. The so-called "micropore volume" refers to the volume of all micropores (generally referring to pores with a pore diameter less than 2 nanometers) per unit mass of the catalyst.

In the context of the specification, the so-called "specific surface area" refers to the total area per unit mass of the sample, including the internal surface area and the external surface area. Non-porous samples only have an external surface area, such as Portland cement, some clay mineral powders, etc.; porous samples have an external surface area and an internal surface area, such as asbestos fibers, diatomaceous earth, and molecular sieves, etc. The surface area within the micropores having a pore diameter less than 2 nanometers in porous samples is the internal surface area. The surface area after deducting the internal surface area is called the external surface area. The external surface area of the unit mass sample is the external specific surface area. Correspondingly, the "external surface of the catalyst" mentioned in the present application refers to the surface of the catalyst except the internal surface of micropores with a pore diameter less than 2 nanometers.

In the context of the specification, the "H-type silica-alumina molecular sieve" has the meaning generally understood in the art, and particularly refers to a silica-alumina molecular sieve with active acid sites. It can usually be prepared directly by using an acidic system, or by subjecting alkaline metal-type silica-alumina molecular sieves such as Na-type silica-alumina molecular sieves to ammonium ion exchange and calcining.

In the present application, unless otherwise stated, the amount and content of the metal component with hydrogenation activity are based on metal.

Unless otherwise stated, the terms used herein have the same meanings as commonly understood by those skilled in the art. If the term is defined herein and its definition is different from the common understanding in the art, the definition herein shall prevail.

In the present application, unless expressly stated, any matters not mentioned shall directly apply to those known in the art without any change. Moreover, any embodiment described herein can be freely combined with one or more other embodiments described herein, and the technical solutions or technical ideas formed thereby are regarded as part of the original disclosure or original record of the present application and shall not be regarded as new content that has not been disclosed or expected herein, unless those skilled in the art believe that the combination is obviously unreasonable.

All patent and non-patent literatures mentioned herein, including but not limited to textbooks and journal articles, are hereby incorporated by reference in their entirety.

As mentioned above, in the first aspect, the present application provides a hydrogenation-acid catalysis bifunctional catalyst, based on the mass of the catalyst, containing 80-99.8% of a silica-alumina molecular sieve component, and 0.2-2% of a metal component with hydrogenation activity supported on the molecular sieve and 0-20% hydrocarbyl modifying component.

In the catalyst of the present application, the hydrocarbyl modifying component is connected to the surface of the molecular sieve through covalent bonding, such as covalent bonding of hydrocarbyl-Si-O-molecular sieve. In certain embodiments, a silanization reagent can be used as a raw material, and the hydrocarbyl modifying component can be connected to the surface of the molecular sieve through the reaction of the silicon-oxygen bonds connected to silicon with the active hydroxyl groups on the surface of the molecular sieve.

In a preferred embodiment, the hydrogenation active metal is selected from ruthenium, platinum, palladium, copper, nickel, or a combination thereof, more preferably ruthenium, palladium, or a combination thereof.

In a preferred embodiment, the hydrocarbyl modifying component is a $C_{1-20}$ hydrocarbyl, preferably a $C_{1-10}$ hydrocarbyl, more preferably selected from methyl, ethyl, propyl, isopropyl, butyl, phenyl, benzyl, phenethyl, or a combination thereof.

In a preferred embodiment, in the catalyst, based on the mass of the catalyst, the mass content of the silica-alumina molecular sieve is 90-99.8%, such as 91%, 92%, 93%, 94%, 95%, 96%, 97%, 98%, 99%, 99.5% and so on. Further preferably, the mass content of the silica-alumina molecular sieve is 90-98%.

In a preferred embodiment, in the catalyst, based on the mass of the catalyst, the mass content of the hydrogenation active metal is 0.2-1.5%, preferably 0.2-1.2%, more preferably 0.3-1.0%, for example 0.4%, 0.5%, 0.6%, 0.7%, 0.8%, 0.9%, etc.

In a preferred embodiment, in the catalyst, the silica-alumina molecular sieve is selected from molecular sieve with MWW, FAU, MOR, BEA or ATS structure, or a combination thereof, preferably molecular sieve with ATS structure. As specific examples of the molecular sieve with MWW structure, SCM-1 molecular sieve, MCM-22 molecular sieve, etc. can be mentioned; as specific examples of the molecular sieve with FAU structure, X molecular sieve and Y molecular sieve can be mentioned; as specific examples of the molecular sieve with MOR structure, LZ-211 molecular sieve can be mentioned; as specific examples of the molecular sieve with BEA structure, beta molecular sieve can be mentioned. In certain particular embodiments, the silica-alumina molecular sieve is H-type silica-alumina molecular sieve.

In a preferred embodiment, the silicon-aluminum ratio of the molecular sieve in the silica-alumina catalyst is 2-50, preferably 2-40, and more preferably 2-20.

In certain preferred embodiments, in the catalyst, the molecular sieve is a silica-alumina molecular sieve with ATS structure, and the X-ray diffraction spectrum of the catalyst shows the relative intensity characteristics of the diffraction peaks as shown in the following table:

| $2\theta(°)$ | Relative intensity ($I/I_0 \times 100$) |
|---|---|
| 7.588-8.188 | vs |
| 16.286-16.886 | m-s |
| 18.927-19.527 | s |
| 20.492-21.092 | s |
| 22.096-22.696 | m-s |
| 26.983-27.583 | m-s |

In a further preferred embodiment, the X-ray diffraction spectrum of the catalyst shows the relative intensity characteristics of the diffraction peaks shown in any row of the following table:

| $2\theta(°)$ | Relative intensity ($I/I_0 \times 100$) |
|---|---|
| 21.600-22.200 | m |
| 28.224-28.824 | w-m |
| 28.975-29.575 | w-m |
| 30.255-30.855 | w-m |
| 31.794-32.394 | m |
| 34.607-35.207 | m |

In a further preferred embodiment, in the catalyst, the crystals have a strip-like or rod-like morphology, the length of the crystals is 0.3-3 $\mu$m, and the aspect ratio is 2-20, preferably 5-20.

According to the present application, the catalyst may optionally undergo one or both of hydrocarbylation treatment and reduction treatment, wherein the hydrocarbylation treatment and the reduction treatment may be carried out during the preparation of the catalyst after supporting the hydrogenation active metal, or they can be carried out before the use of the catalyst; and, when both the hydrocarbylation treatment and the reduction treatment are carried out, the reduction treatment can be carried out before or after the hydrocarbylation treatment, preferably after the hydrocarbylation treatment. In a preferred embodiment, the catalyst undergoes the reduction treatment. In addition, when the silica-alumina molecular sieve is a molecular sieve having MWW, FAU, MOR or BEA structure, the catalyst is preferably subjected to hydrocarbylation treatment.

According to the present application, in the catalyst, the hydrogenation active metal may be present in various forms, such as a form selected from metal element, oxide, chloride, nitrate, or a combination thereof. In a preferred embodiment, the hydrogenation active metal mainly exists in the form of metal element, for example, when the catalyst undergoes reduction treatment. Preferably, in the catalyst, the particle size of the hydrogenation active metal particles is 0.5-10 nm, preferably 1-5 nm.

In certain preferred embodiments, in the catalyst, the silica-alumina molecular sieve is a molecular sieve with ATS structure, especially ATS molecular sieve with the above XRD spectrum characteristics, and based on the mass of the catalyst, the mass content of the silicon-aluminum molecular sieve is 80-99.8%, preferably 90-99.8%, the mass content of the hydrogenation active metal is 0.2-2%, preferably 0.2-1.5%, and the catalyst does not contain the hydrocarbyl modifying component.

In other preferred embodiments, especially when the silica-alumina molecular sieve is a molecular sieve with MWW, FAU, MOR or BEA structure, in the catalyst, based on the mass of the catalyst, the mass content of the silica-alumina molecular sieve is 80-98%, preferably 90-98%, the mass content of the hydrogenation active metal is 0.2-1.5%, preferably 0.2-1.2%, more preferably 0.3-1.0%, and the mass content of the hydrocarbyl modifying component is 1-20%, preferably 1-10%, more preferably 2-10%, such as 2%, 3%, 4%, 5%, 6%, 7%, 8%, 9%, etc.

In a further preferred embodiment, as tested by X-ray photoelectron spectroscopy (XPS), the mass content of the hydrogenation active metal on the external surface of the catalyst to the elements on the external surface is 0.5% or less, preferably 0.4% or less, and more preferably 0.01-0.35%, such as 0.01-0.2%.

In a further preferred embodiment, the distribution coefficient of the hydrogenation active metal on the external surface of the catalyst is 1-20%, preferably 1.5-18%, more preferably 1.5-12%, such as 1.5-10%.

In the present application, the distribution coefficient of the hydrogenation active metal on the external surface of the catalyst is calculated by the following formula :

Distribution coefficient of hydrogenation active metal on the external surface = ( mass content of the hydrogenation active metal on the external surface of the catalyst to the elements on the external surface * external specific surface area of the catalyst ) / ( total mass content of hydrogenation active metal in the catalyst * specific surface area of the catalyst) * 100%, wherein the mass content of the hydrogenation active metal on the external surface of the catalyst to the elements on the external surface is measured by X-ray photoelectron spectroscopy (XPS), and the total mass content of hydrogenation active metal in the catalyst can be measured by inductively coupled plasma atomic emission spectroscopy (ICP).

According to the present application, the distribution coefficient of the hydrogenation active metal on the external surface of the catalyst can be used to characterize the proportion of the hydrogenation active metal content on the external surface of the catalyst to the total hydrogenation active metal content. Specifically, when the hydrogenation active metal in the catalyst is supported on the internal and external surfaces of the molecular sieve using a simple impregnation method, the proportion of the hydrogenation active metal content on the external surface to the total hydrogenation active metal content is not only proportional to the relative distribution of the hydrogenation active metal on the external surface, but also proportional to the proportion of the catalyst external surface area.

In a preferred embodiment, the specific surface area of the catalyst is 200-800 $m^2/g$, preferably 250-700 $m^2/g$, such as 300 $m^2/g$, 380 $m^2/g$, 385 $m^2/g$, 410 $m^2/g$, 490 $m^2/g$ or 500 $m^2/g$.

In a preferred embodiment, the total pore volume of the catalyst is not less than 0.15 $cm^3/g$, preferably 0.18-1.0 $cm^3/g$, more preferably 0.2-1.0 $cm^3/g$, such as 0.2-0.9 $cm^3/g$.

In a preferred embodiment, the micropore volume of the catalyst is 0.05-0.30 $cm^3/g$, preferably 0.10-0.25 $cm^3/g$.

In a preferred embodiment, the total acid content of the catalyst is 400-1500 $\mu mol \cdot g^{-1}$, preferably 600-1500 $\mu mol \cdot g^{-1}$, for example, 800 $\mu mol \cdot g^{-1}$, 1250 $\mu mol \cdot g^{-1}$, 1300 $\mu mol \cdot g^{-1}$ or 1500 $\mu mol \cdot g^{-1}$.

In some preferred embodiments, for example, when the catalyst undergoes hydrocarbylation treatment (that is, when the content of the hydrocarbyl modifying component in the catalyst is in the range of 1-20%, preferably 1-10%), the relative acid equivalent of the external surface of the catalyst is 15-50%, preferably 15-40%, such as 20%, 30% or 35%.

In certain preferred embodiments, for example, when the catalyst undergoes hydrocarbylation treatment, the acid content ratio of B acid/L acid of the catalyst is 0.2-8.0, preferably 0.4-6.0, more preferably 3.0-6.0, such as 5.0 or 5.5.

In other preferred embodiments, for example, when the catalyst is based on ATS molecular sieve and has not undergone hydrocarbylation treatment, the acid content ratio of B acid/L acid of the catalyst is 3-10, preferably 5-7.

In a preferred embodiment, in the case of hydrocarbylation treatment but no reduction treatment, the metal $H_2$-TPR test reduction temperature of the catalyst is 470-500°C, preferably 480-500°C.

In a second aspect, a method for preparing the catalyst of the present application is provided, comprising the following steps:

(1) providing H-type silica-alumina molecular sieve; and
(2) supporting the hydrogenation active metal on the H-type silica-alumina molecular sieve, and optionally performing hydrocarbylation treatment and/or reduction on the resulting product to obtain the catalyst.

In a preferred embodiment, the H-type silica-alumina molecule is selected from molecular sieve with MWW, FAU, MOR, BEA or ATS structure, or a combination thereof, preferably molecular sieve with ATS structure.

In some preferred embodiments, step (1) includes subjecting the silica-alumina molecular sieve raw material to ammonium ion exchange and calcining to obtain the H-type silica-alumina molecular sieve.

In a further preferred embodiment, the silica-alumina molecular sieve raw material is selected from silica-alumina molecular sieve with MWW, FAU, MOR or BEA structure, or a combination thereof.

In certain specific embodiments, in step (1), the ammonium ion exchange is to exchange alkali metal or alkaline earth metal cations such as $Na^+$ and $K^+$ in the alkaline metal type molecular sieve into $NH^{4+}$ at 20-60 °C for 0.5-4h, and can perform once or multiple times. The ammonium salt in the ammonium ion exchange is selected from one or more of ammonia, ammonium chloride, ammonium nitrate, and ammonium carbonate. The concentration of the ammonium salt is 0.1-1.0 mol/L. After ammonium ion exchange, the molecular sieve is dried at 60-120 °C for 4-24h, and then calcined. The calcining temperature is 400-650 °C, the calcining time is 1-12 hours, and the calcining atmosphere is oxygen or air to obtain the H-type silica-alumina molecular sieve.

In other preferred embodiments, step (1) includes mixing a silicon source, an aluminum source, a fluorine source, an organic structure directing agent and water, and after heating pretreatment, crystallization treatment and calcining are performed to obtain H-type ATS silica-alumina molecular sieve.

In a further preferred embodiment, in step (1), the molar ratio of the added silicon source, calculated as $SiO_2$, the aluminum source, calculated as $Al_2O_3$, the fluorine source, calculated as $F^-$, the organic structure directing agent and water is 1: (0.02-0.2): (0.5-2): (0.25-1.5): (3-15), preferably 1: (0.05-0.15): (0.5-1): (0.5-1): (5-10).

In a further preferred embodiment, in step (1), the silicon source is selected from silicic acid, silica gel, silica sol, tetraethyl silicate, sodium silicate, or a combination thereof, and the aluminum source is selected from pseudo-boehmite, aluminum isopropoxide, or a combination thereof, the fluorine source is hydrofluoric acid, and the organic structure directing agent is 4-pyrrolidinylpyridine. In this embodiment, 4-pyrrolidinylpyridine is used as the organic structure directing agent. There is no need to add a base during the reaction, and the obtained molecular sieve can be used as a catalyst without ammonium ion exchange.

In a further preferred embodiment, in step (1), the heating pretreatment method is rotary evaporation to remove water or open heating to remove water, and the processing condition of the open heating is heating and stirring at 50-100 °C, preferably heating and stirring at 70-90 °C.

In a further preferred embodiment, in step (1), after the raw material mixture is heated and pretreated, the molar ratio of the silicon source (calculated as $SiO_2$) to water during crystallization is 1: (1-10), preferably 1: (1.5-6.5).

In a further preferred embodiment, in step (1), the crystallization conditions include: crystallization temperature is 120-200 °C, preferably 150-200 °C, and crystallization time is 7-21 days, preferably 7-15 days.

In a further preferred embodiment, in step (1), the crystallization can be carried out in any manner conventionally known in the art, for example, the silicon source, the aluminum source, the fluorine source, the organic structure directing agent and water are mixed in a predetermined ratio and the resulting mixture is heated and crystallized under crystallization conditions.

In a further preferred embodiment, in step (1), after the crystallization step is completed, the product can be obtained from the mixture obtained by any conventionally known separation method and calcining treatment. Examples of the separation method include filtering, washing and drying the obtained mixture. Here, filtering, washing and drying may be performed in any manner conventionally known in the art. For example, as filtering, the obtained product mixture may be simply suction filtered. Examples of the washing include washing with deionized water and/or ethanol. Examples of the drying temperature include 40-250 °C, preferably 60-150 °C, and examples of the drying time include 8-30 hours, preferably 10-20 hours. This drying may be performed under normal pressure or under reduced pressure.

In a further preferred embodiment, in step (1), the calcining can be carried out in any manner conventionally known in the art. For example, the calcining temperature is generally 300-800 °C, preferably 400-650 °C, and the calcining time is generally 1-12 hours, preferably 2-6 hours. In addition, the calcining is generally performed in an oxygen-containing atmosphere, such as air or oxygen atmosphere.

According to the present application, in step (2), the hydrogenation active metal can be supported onto the H-type silica-alumina molecular sieve through conventional means (such as impregnation). In a preferred embodiment, step (2) supports the hydrogenation active metal onto the H-type silica-alumina molecular sieve by adding a solution of the hydrogenation active metal source to the H-type silica-alumina molecular sieve and drying.

In a further preferred embodiment, in step (2), the hydrogenation active metal source is selected from a soluble compound of the metal, preferably selected from a chloride, a nitrate of the metal, or a combination thereof. Taking ruthenium as an example, the solution of the hydrogenation active metal source may be a ruthenium-containing solution prepared from ruthenium nitrate or ruthenium chloride.

In a further preferred embodiment, in step (2), based on the mass of the hydrogenation active metal, the concentration of the solution of the hydrogenation active metal source is 1.5-50g/L, preferably 2-45g/L.

In a further preferred embodiment, in step (2), the solution of the hydrogenation active metal source is added dropwise to the H-type silica-alumina molecular sieve of step (1). The present application does not specifically limit the dropwise adding conditions. For example, it can be added dropwise at room temperature and then mixed for 1 to 10 hours.

In a further preferred embodiment, in step (2), the mass ratio of the hydrogenation active metal in the solution of the hydrogenation active metal source to the H-type silica-alumina molecular sieve in step (1) is 0.002-0.015: 1, for example 0.005-0.02:1.

In a further preferred embodiment, in step (2), the drying can be performed in a conventional manner, such as oven drying. The drying conditions preferably include: the drying temperature is 40-90 °C, and the drying time is 4-12 hours.

According to the present application, in step (2), after supporting the hydrogenation active metal on the H-type silica-alumina molecular sieve, the obtained product can optionally be subjected to one or both of hydrocarbylation treatment and reduction treatment, and when both hydrocarbylation treatment and reduction treatment are performed, the reduction treatment may be performed before or after the hydrocarbylation treatment, preferably after the hydrocarbylation treatment.

In a preferred embodiment, step (2) includes reducing the product after supporting the hydrogenation active metal. Further preferably, the reduction can be carried out using reducing gas, preferably hydrogen reduction, and the reduction conditions preferably include: the reduction temperature is 300-550 °C, the reduction time is 3-6 hours, and the volume space velocity of the reducing gas is 40-200h$^{-1}$.

In a preferred embodiment, especially when the H-type silica-alumina molecular sieve is a molecular sieve with MWW, FAU, MOR or BEA structure, step (2) includes performing the hydrocarbylation treatment on the product after supporting the hydrogenation active metal, preferably, the product after supporting the hydrogenation active metal is first subjected to the reduction treatment and then to the hydrocarbylation treatment.

In a further preferred embodiment, the hydrocarbylation treatment in step (2) includes mixing and reacting the product supported with the hydrogenation active metal, preferably after reduction, with a hydrocarbylation reagent in a solvent.

In a further preferred embodiment, the hydrocarbylation reagent is selected from methyltrimethoxysilane, dimethyldimethoxysilane, ethyltrimethoxysilane, diethyldimethoxysilane, propyltrimethoxylsilane, isopropyltrimethoxysilane, phenyltrimethoxysilane, tolyltrimethoxysilane, phenylsilanetriol, tolylsilanetriol, diphenylsilanediol, or a combination thereof, preferably selected from dimethyldimethoxysilane, diethyldimethoxysilane, isopropyltrimethoxysilane, phenyltrimethoxysilane, tolyltrimethoxysilane, phenylsilanetriol, tolylsilanetriol, or a combination thereof. Further preferably, the solvent used in the hydrocarbylation treatment is selected from ethanol, toluene or a combination thereof.

In a further preferred embodiment, in the hydrocarbylation treatment, the mass ratio of the product supported with the hydrogenated active metal, the hydrocarbylation reagent and the solvent is 1: (0.05-0.45): (5-55), preferably 1: (0.06-0.40): (6-50), for example, 1: (0.10-0.33): (8-50) or 1: (0.12-0.35): (7.5-52).

In a further preferred embodiment, in the hydrocarbylation treatment, the reaction conditions include: the reaction temperature is 40-110 °C, preferably 70-110°C, and the reaction time is 6-48h, preferably 8-24 hours.

In certain further preferred embodiments, after the hydrocarbylation treatment, the resulting reaction product is separated (for example filtered), washed and dried. According to the present application, the separation, washing and drying may be carried out in any manner conventionally known in the art. For example, as filtering, the obtained product mixture may be simply suction filtered. Examples of the washing include washing with deionized water and/or ethanol. Examples of the drying temperature include 40-250 °C, preferably 60-150 °C, and examples of the drying time include 8-30 hours, preferably 10-20 hours. The drying may be performed under normal pressure or under reduced pressure.

In a third aspect, a hydrogenation-acid catalysis bifunctional catalyst prepared by the method of the present application is provided.

In a specific embodiment, various characteristics of the hydrogenation-acid catalysis bifunctional catalyst obtained by the method of the present application are as described in the first aspect of the present application, and will not be described again here.

In a fourth aspect, the application of the catalyst of the present application in hydrocarbon hydroconversion reaction is provided, including the step of contacting and reacting the hydrocarbon feedstock with the catalyst in the presence of hydrogen.

In a preferred embodiment, the hydrocarbon hydroconversion reaction is selected from benzene hydroalkylation reaction and alkane hydroisomerization reaction.

In a fifth aspect, the present application provides a one-step method for producing cyclohexylbenzene by hydrogenating benzene, which includes contacting and reacting benzene with the catalyst described in the present application in the presence of hydrogen to obtain cyclohexylbenzene.

In a preferred embodiment, the reaction conditions include: the mass ratio of benzene to the catalyst is 8-40, preferably 10-40; the reaction temperature is 100-220 °C, preferably 120-200 °C; the reaction time is 2-8 hours, preferably 2.5-6 hours; the hydrogen pressure is 0.8-2.5MPa, preferably 1.0-2.5MPa.

In a sixth aspect, the present application provides an alkane hydroisomerization method, which includes contacting and reacting linear alkane with the catalyst of the present application in the presence of hydrogen to obtain an isomerization product, wherein the linear alkane is a C8 or higher linear alkane, preferably a C8-C20 linear alkane, and more preferably a C8-C12 linear alkane, such as n-heptane, n-decane, etc.

In a preferred embodiment, the reaction conditions include: the mass ratio of the linear alkane to the catalyst is 10-100, preferably 10-50; the reaction temperature is 250-400°C, preferably 300-400°C; the reaction time is 3-10 hours, preferably 4-10 hours; the hydrogen pressure is 2.5-5.0MPa, preferably 3.0-4.0MPa.

In certain preferred embodiments, the following technical solutions are provided:

A1. A catalyst for producing cyclohexylbenzene, which has a schematic chemical composition as shown in the formula "$xM \cdot ySiO_2 \cdot zAl_2O_3$";

wherein, M is a metal element selected from one or more of ruthenium, platinum, palladium, copper and nickel metals; wherein, in the chemical composition, $0.001 \leq x/y \leq 0.02$, $8 \leq y/z \leq 80$.

A2. The catalyst according to item A1, characterized in that, in the catalyst, based on the mass of the catalyst, the mass content of metal M is 0.2%-2%.

A3. The catalyst according to item A1, characterized in that the catalyst has an X-ray diffraction pattern as shown in the following table,

| $2\theta(°)$ | Relative intensity ($I/I_0 \times 100$) |
| --- | --- |
| 7.888±0.3 | vs |
| 16.586±0.3 | m-s |
| 19.227±0.3 | s |
| 20.792±0.3 | s |
| 22.396±0.3 | m-s |
| 27.283±0.3 | m-s |

A4. The catalyst according to item A1 or A3, characterized in that the catalyst also includes an X-ray diffraction pattern as shown in the following table,

| $2\theta$ (°) [a] | Relative intensity ($I/I_0 \times 100$) |
| --- | --- |
| 21.900±0.3 | m |
| 28.524±0.3 | w-m |
| 29.275±0.3 | w-m |
| 30.555±0.3 | w-m |
| 32.094±0.3 | m |
| 34.907±0.3 | m |

A5. The catalyst according to item A1, characterized in that the total acid content of the catalyst is 500-1500 $\mu$mol·g$^{-1}$, preferably 800-1500 $\mu$mol·g$^{-1}$, and the acid content ratio of B acid/L acid is 3-10, preferably 5-7.

A6. The catalyst according to item A1, characterized in that in the catalyst, the crystals have a strip-like or rod-like morphology, the length of the crystal is 0.3-3 $\mu$m, and the aspect ratio is 2-20.

A7. The catalyst according to item A1, characterized in that the specific surface area of the catalyst is 200-600 m$^2$/g, preferably 250-500 m$^2$/g; the micropore volume of the catalyst is 0.05-0.30 cm$^3$/g, preferably 0.10-0.25 cm$^3$/g.

A8. A method for preparing the catalyst for producing cyclohexylbenzene according to any one of items A1-7, comprising the following steps:

(1) mixing silicon source, aluminum source, fluorine source, organic structure directing agent and water, and after heating pretreatment, crystallizing and calcining to obtain sample B;

(2) adding a solution containing metal M to the sample B of step (1), and then drying and reducing to prepare the catalyst.

A9. The preparation method according to item A8, characterized in that in step (1), the molar ratio of the added silicon source, calculated as $SiO_2$, the aluminum source, calculated as $Al_2O_3$, the fluorine source, calculated as $F^-$, the organic structure directing agent and water is 1: (0.02-0.2): (0.5-2): (0.25-1.5): (3-15), preferably 1: (0.05-0.15): (0.5-1): (0.5-1): (5-10).

A10. The preparation method according to item A8 or A9, characterized in that in step (1), the silicon source is selected from at least one of silicic acid, silica gel, silica sol, tetraethyl silicate, and sodium silicate; the aluminum source is selected from at least one of pseudo-boehmite and aluminum isopropoxide; in step (1), the fluorine source is hydrofluoric acid; the organic structure directing agent is 4-pyrrolidinylpyridine.

A11. The preparation method according to item A8 or A9, characterized in that in step (1), after the raw material mixture undergoes heating pretreatment, the molar ratio of the silicon source, calculated as $SiO_2$, to water during crystallization is 1: (1-10), preferably 1: (1.5-6.5).

A12. The preparation method according to item A8 or A9, characterized in that in step (1), the crystallization conditions include: the temperature is 120-200 °C, the time is 7-21 days, preferably, the temperature is 150 -200 °C, the time is 7-15 days.

A13. The preparation method according to item A8 or A9, characterized in that in step (2), the concentration of the solution containing metal M is 2-50g/L.

A14. A one-step method for producing cyclohexylbenzene by hydrogenating benzene by using the catalyst described in any one of items A1 to A7.

A15. The method according to item A14, characterized in that the method includes a contact reaction between the raw material benzene and the catalyst, using hydrogen as the hydrogen source to produce cyclohexylbenzene; wherein the mass ratio of the raw material benzene to the catalyst is 8-40; the reaction temperature is 100-220 °C, the reaction time is 2-8 hours, and the hydrogen pressure is 0.8-2.5MPa.

B1. A catalyst for producing cyclohexylbenzene, characterized in that the catalyst includes molecular sieve, active metal M and hydrocarbyl modifying group;

wherein, the active metal M is selected from one or more of ruthenium, platinum, palladium, copper and nickel; wherein, the hydrocarbyl modifying group is selected from at least one of C1-C4 alkyl groups; wherein, based on the mass of the catalyst, the mass content of the active metal M is 0.2%-1.5%; the mass content of the metal M on the external surface of the catalyst to the external surface elements is 0.4% or less; the distribution coefficient of the metal M on the external surface is 1.2%-20%.

B2. The catalyst according to item B1, characterized in that, in the catalyst, based on the mass of the catalyst, the mass content of the metal M is 0.2%-1.2%.

B3. The catalyst according to item B1, characterized in that, in the catalyst, based on the mass of the catalyst, the mass content of the hydrocarbyl modifying group is 1%-10%.

B4. The catalyst according to item B1, characterized in that the molecule sieve in the catalyst is selected from at least one of MWW, FAU, MOR, BEA, and ATS molecular sieves; the molecular sieve in the catalyst accounts for 90%-98% of the catalyst mass, the silicon to aluminum ratio is 2-50, preferably 4-40.

B5. The catalyst according to item B1, characterized in that the specific surface area of the catalyst is 380-800m²/g, preferably 400-700m²/g; the total pore volume of the catalyst is not less than 0.15cm³/g, preferably 0.2-0.9cm³/g.

B6. A method for preparing the catalyst for producing cyclohexylbenzene according to any one of items B1 to B5, comprising the following steps :

(i) subjecting the molecular sieve raw material to ammonium ion exchange and calcining to obtain the H-type molecular sieve;

(ii) adding a solution containing metal M to the H-type molecular sieve of step (1), and obtaining a catalyst precursor through drying and reduction;

(iii) mixing and reacting the catalyst precursor, alkylating reagent and solvent, and then filtering, washing and drying to prepare the catalyst.

B7. The preparation method according to item B6, characterized in that in step (i), the concentration of the solution containing metal M is 2-50g/L.

B8. The preparation method according to item B6, characterized in that in step (ii), the alkylating reagent is selected from one or more of methyltrimethoxysilane, dimethyldimethoxysilane, ethyltrimethoxysilane, diethyldimethoxysilane, propyltrimethoxysilane, isopropyltrimethoxysilane, preferably one or more of dimethyldimethoxysilane, diethyldimethoxysilane and isopropyltrimethoxysilane; the solvent is at least one of ethanol or toluene.

B9. The preparation method according to item B 6, characterized in that in step (iii), the mass ratio of the added catalyst precursor, alkylating reagent and solvent is 1: (0.05-0.40): (5-50).

B10. A one-step method for producing cyclohexylbenzene by hydrogenating benzene by using the catalyst described in any one of items B1 to B5.

C1. A one-step method for producing cyclohexylbenzene by hydrogenating benzene, including a contact reaction between the raw material benzene and the catalyst, using hydrogen as the hydrogen source to produce cyclohexylbenzene; the catalyst includes molecular sieve, active metal M and hydrocarbyl modifying group;

> wherein, the active metal M is selected from one or more of ruthenium, platinum, palladium, copper and nickel;
> wherein, the hydrocarbyl modifying group is selected from at least one of phenyl, benzyl, and phenethyl;
> wherein, the total acid content of the catalyst is 400-1500 $\mu$mol·g$^{-1}$, and the relative acid equivalent of the external surface of the catalyst is 15%-35%.

C2. The method according to item C1, characterized in that the metal M is preferably ruthenium or/and palladium metal element.

C3. The method according to item C1, characterized in that, in the catalyst, based on the mass of the catalyst, the mass content of the hydrocarbyl modifying group is 2%-20%.

C4. The method according to item C1, characterized in that the molecular sieve in the catalyst is selected from at least one of MWW, FAU, MOR, BEA, and ATS molecular sieves; the molecular sieve in the catalyst accounts for 80%-95% of the catalyst mass, the molar ratio of silicon to aluminum is 2-50, preferably 4-40.

C5. The method according to item C1, characterized in that the specific surface area of the catalyst is 385-500m$^2$/g, preferably 410-490m$^2$/g; the total pore volume of the catalyst is not less than 0.18cm$^3$/ g, preferably 0.18-1.0cm$^3$/g.

C6. The method according to item C1, characterized in that the preparation method of the catalyst includes the following steps:

> (A) mixing a solution containing metal M with H-type molecular sieve, and then drying and reducing to prepare a catalyst precursor;
> (B) mixing and reacting the catalyst precursor, arylation reagent and solvent, and then filtering, washing and drying to prepare the catalyst.

C7. The method according to item C6, characterized in that in step (A), the concentration of the solution containing metal M is 1.5-45g/L.

C8. The method according to item C6, characterized in that in step (B), the arylation reagent is selected from one or more of phenyltrimethoxysilane, tolyltrimethoxysilane, phenylsilanetriol, tolylsilanetriol and diphenylsilanediol; the solvent is at least one of ethanol or toluene.

C9. The method according to item C6, characterized in that in step (B), the mass ratio of the added catalyst precursor, the arylation reagent and the solvent is 1: (0.06-0.45): (6-55).

C10. The method according to item C1, characterized in that in the reaction, the mass ratio of the raw material benzene to catalyst is 8-40, the reaction temperature is 100-220 °C, the reaction time is 2-8 hours, and the hydrogen pressure is 0.8-2.5MPa.

**Examples**

The technical solutions of the present application will be further described in detail through the examples below, but the protection scope of the present application is not limited to these examples.

In the following examples and comparative examples, unless otherwise specified, all reagents and raw materials used are commercially available products, and their purity is of analytical grade.

Experimental methods that do not indicate specific conditions in the following examples and comparative examples should be selected according to conventional methods and conditions, or according to product instructions.

In the present application, including in the following examples and comparative examples, the structure of the sample is determined by the X-ray diffraction spectrum (XRD), and the XRD spectrum is determined by an X-ray powder diffractometer. The model of the X-ray powder diffractometer used is Panalytical X PERPRO X-ray powder diffractometer. The following conditions are used to analyze the physical phase of the sample: CuK$\alpha$ ray source ($\lambda$=1.54Å), nickel filter, 2$\theta$ scanning range 2-50°, operating voltage 40kV, current 40mA, scanning rate 10°/min.

In the present application, including in the following examples and comparative examples, the model of the scanning electron microscope (SEM) used is S-4800II field emission scanning electron microscope. The method of measuring the crystal particle size of the sample is: using the scanning electron microscope to observe the molecular sieve at a magnification of 10,000 times, randomly selecting an observation field of view, calculating the average of the sum of the particle sizes of all crystals in the observation field of view, and repeating the operation for 10 times in total. The average value of the sum of 10 average values is used as the crystal particle size.

In the present application, including in the following examples and comparative examples, the sample size is measured by: using a transmission electron microscope (G2F30 transmission electron microscope, FEI Company of the Netherlands, working voltage 300kV) to observe the molecular sieve at a magnification of 100,000 times, randomly selecting an observation field of view, calculating the average of the sum of the sizes of all particles in the observation field of view, repeating this operation for 10 times in total, and using the average value of the sum of 10 average values as the size of the particles.

In the present application, including in the following examples and comparative examples, the acid content and acid type of the sample are measured using the pyridine adsorption infrared method (Nicolet Model 710 spectrometer). The specific operating steps are as follows: a. sample pretreatment. The sample (about 30 mg) is pressed into thin disk with a diameter of 13 mm and put into the infrared sample tank. Afterwards, the sample is pretreated under vacuum cell conditions and 400 °C for 1 h. After the sample tank cools to room temperature, scan the infrared data of the sample as the background. b. Pyridine adsorption. At room temperature and in a vacuum environment, pyridine vapor is introduced into the original position until the adsorption reached equilibrium, and the adsorption time is 1 h. c. Pyridine desorption. After the adsorption is completed, evacuate at 100 °C until the internal pressure no longer changes. The desorption time is 40 minutes, and the infrared absorption spectra are scanned and recorded respectively. The difference spectrum before and after pyridine adsorption is the resulting pyridine adsorption-infrared absorption spectrum. Calculate the acid content of the sample based on the spectrum:

$$C_L = K_L \times A_{1440} = \frac{\pi}{IMEC_L} \times \left(\frac{r^2}{w}\right) \times A_{1440} \qquad C_B = K_B \times A_{1540} = \frac{\pi}{IMEC_B} \times \left(\frac{r^2}{w}\right) \times A_{1540}$$

wherein, r and w are the diameter (cm) and mass (g) of the catalyst thin disk, and A is the integrated absorbance value at the specified wave number peak based on the scanning pyridine adsorption-infrared absorption spectrum. IMEC is the integrated molar extinction coefficient, $IMEC_L$ is 2.22, and $IMEC_B$ is 1.67. The peak near 1545cm$^{-1}$ is B acid, and the peak near 1455cm$^{-1}$ is L acid.

In the present application, including in the following examples and comparative examples, the characterization of the relative acid equivalent on the external surface of the catalyst is measured using the "probe reaction" triisopropylbenzene pyrolysis. The specific operation is to prepare a chromatographic column sample of 50 mg of catalyst mixed with 100 mg of quartz sand, and then 1 $\mu$L of triisopropylbenzene liquid is injected at a time through gas chromatography (GC, Agilent 7890B) at 250°C. Then, according to the yield of cyclohexene in the chromatogram, compare the structure of "metal-molecular sieve" without hydrocarbylation treatment to evaluate the relative acid content and activity of the external surface of the catalyst. The specific calculation method is as follows:

Relative acid equivalent on the external surface = (propylene output of the hydrocarbylation group/(3×triisopropylbenzene addition amount of the hydrocarbylation group))/(propylene output of the unhydrocarbylation group/(3×triisopropylbenzene addition amount of the unhydrocarbylation group) )×100%.

In the present application, including in the following examples and comparative examples, the total pore volume, micropore volume, total specific surface area and external specific surface area of the sample are measured by the nitrogen physical adsorption and desorption method (BET method): using a physical adsorption instrument (such as Micromeretic ASAP2020M physical adsorption instrument) to measure the nitrogen physical adsorption and desorption isotherm of the molecular sieve, and then calculating using the BET equation and the t-plot equation.

In the present application, including in the following examples and comparative examples, the model of the inductively coupled plasma atomic emission spectrometer (ICP) used is Varian 725-ES. The analyzed sample is dissolved with hydrofluoric acid to detect the element content (in mole), including the hydrogenation active metal in the sample (hereinafter referred to as "metal M"), and then the mass content is obtained through conversion.

In the present application, including in the following examples and comparative examples, the mass content of the metal M on the external surface of the catalyst to the external surface elements is determined according to the X-ray photoelectron spectroscopy (XPS) test of the catalyst surface element state. The model of the X-ray photoelectron spectrometer used is the ESCA LAB-250 X-ray photoelectron spectrometer of Thermo Company, which uses Al Ka=1486.6eV as the X-ray source, the working voltage is 12 KV, and the working current is 20 mA, the detection thickness is 3nm, and C1s=284.6eV is used as the internal standard to correct the measured element signals.

In the present application, including in the following examples and comparative examples, the distribution coefficient the metal M on the external surface of the catalyst is calculated by the following formula:

Distribution coefficient of the metal M = ( mass content of the metal M on the external surface of the catalyst to the elements on the external surface * external specific surface area of the catalyst ) / ( total mass content of the metal M in the catalyst * specific surface area of the catalyst ) * 100%.

In the present application, including in the following examples and comparative examples, the metal reduction temperature is obtained by using $H_2$ -TPR test (hydrogen temperature programmed reduction). The TPR tester is AMI-3300 temperature-programmed adsorption instrument of Altamira Instruments Company. The test method is to load the sample and purge it with argon at 300°C for 1 hour, then cool it to 50°C, then introduce a 10% $H_2$-Ar mixed gas with a total flow rate of 30mL/min, and then raise the temperature at 10°C/min to 900°C, measure the $H_2$ consumption curve, and record the peak temperature of the curve as the sample reduction temperature.

In the present application, including in the following examples and comparative examples, the mass content of the hydrocarbyl modifying component is measured using thermogravimetric-mass spectrometry (TG-MS) mass loss ratio, and mass spectrometry is used to confirm the type of modifying hydrocarbyl. The analyzer is the STA449F3-QMS403 model of Netzsch Company. The thermogravimetric results of the sample at 25 -1000°C are measured at a heating rate of 10° C/min.

In the present application, including in the following examples and comparative examples, a Fourier transform infrared spectrometer (FTIR) is used to measure the infrared absorption spectrum of the catalyst. The analyzer is the Nicolet 5700 model from Thermofisher Scientific Company. The test range is the absorption spectrum at wave number 400-4000cm$^{-1}$.

In the present application, including in the following examples and comparative examples, the reaction product cyclohexylbenzene is characterized by gas chromatography-mass spectrometry (GC-MS), and the yield of the product cyclohexylbenzene and the conversion rate of the reaction substrate are analyzed by gas chromatography (GC). The GC/MS instrument is Agilent 7890A from Agilent Company of the United States, the chromatographic column is HP-5 non-polar capillary column (30m, 0.53mm). The gas chromatograph is Agilent 7890B, the detector is a hydrogen flame ionization detector (FID), and the chromatographic column is SE-54 capillary column (30m, 0.53mm).

The calculation formula for the yield and selectivity of the product cyclohexylbenzene are:

The yield % of the product clyclohexylbenzene = (the molars of cyclohexylbenzene produced by the reaction × 2)/(the molars of benzene reacted) × 100%.

The selectivity % of the product cyclohexylbenzene = (the molars of cyclohexylbenzene produced by the reaction × 2) / (the molars of benzene reacted) × 100%

Similarly, in the present application, including in the following examples and comparative examples, for n-decane hydroisomerization reaction, the yield and selectivity formula of the isomerization product are:

The yield % of the isomerization product = (the moles of the isomerization product produced by the reaction)/(the moles of the reaction substrate n-decane) × 100% The selectivity % of the isomerization product = (the moles of the isomerization product produced by the reaction)/(the moles of n-decane reacted) × 100%.

Preparation Example I-1

4g deionized water, 0.75g 4-pyrrolidinylpyridine, 0.42g aluminum isopropoxide, 1.5g silica sol, and 0.5g hydrofluoric acid were mixed uniformly to obtain a component with a composition of $1SiO_2:0.1Al_2O_3:1F^-:29H_2O:0.5OSDA$, followed by open heating pretreatment at 80 °C, and a block mixture was obtained. After the raw material mixture was heated and pretreated, the molar ratio of silicon source (calculated as $SiO_2$) and water during crystallization was 1:5, and then crystallized in a crystallizing kettle at 170 °C for 15 days. After taking out the product, it was washed three times with deionized water, dried and calcined at 550 °C for 6 hours to obtain the required H-type silica-alumina molecular sieve (with ATS structure).

1.2 mL of 3.2 g/L ruthenium chloride solution was added dropwise onto 1 g of the H-type silica-alumina molecular sieve. After drying at 80 °C for 2 hours, it was reduced in a fixed-bed reactor for 3 hours at 350°C and a hydrogen flow rate of 10 mL/min to obtain the required catalyst.

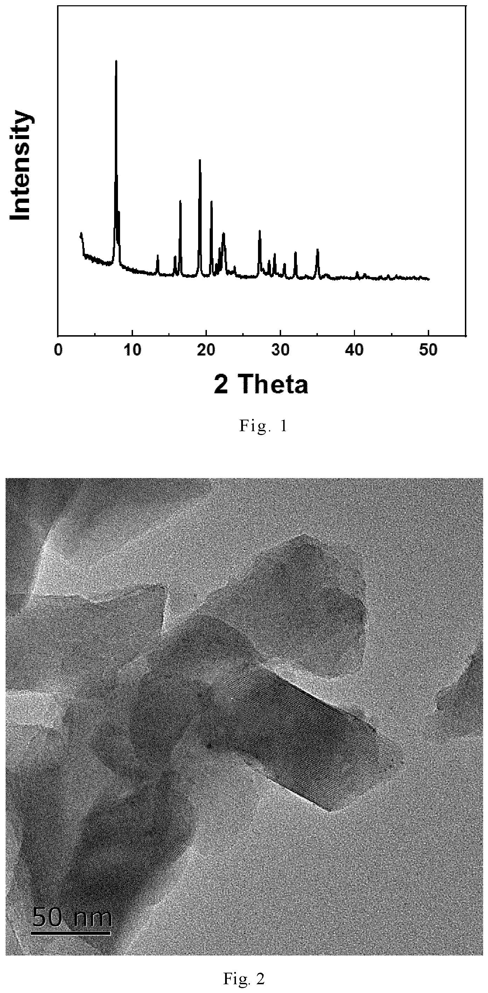

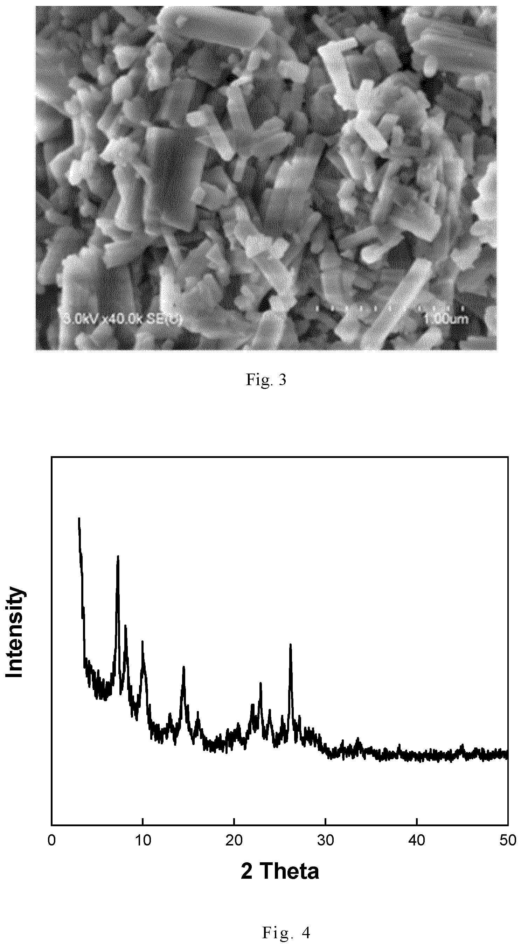

The XRD spectrum data of the catalyst are shown in Table I-1, the XRD spectrum is shown in Figure 1, and the TEM photos and SEM photos are shown in Figures 2 and 3 respectively. In the catalyst composition, $n(SiO_2):n(Al_2O_3)=10$, the Ru mass fraction is 0.3%, and the chemical composition of the catalyst is expressed as "$xM \cdot ySiO_2 \cdot zAl_2O_3$", then x/y=0.0021,y/z=10. The properties of the resulting catalyst are listed in Table I-13.

According to SEM Figure 3, in the catalyst, the crystals have a long strip shape, the length of the crystals is 0.4-1.5 μm, and the aspect ratio is 2-10.

Table I-1 XRD spectrum data of the catalyst obtained in Preparation Example I-1

| $2\theta(°)$ | d-spacing (Å) | Relative intensity ($I/I_0 \times 100$) |
|---|---|---|
| 7.89 | 11.20 | 100 |
| 16.56 | 5.35 | 36 |
| 19.18 | 4.62 | 55 |
| 20.79 | 4.27 | 36 |
| 21.86 | 4.06 | 15 |
| 22.40 | 3.97 | 21 |
| 27.28 | 3.27 | 23 |
| 28.52 | 3.13 | 9 |
| 29.28 | 3.05 | 12 |
| 30.56 | 2.92 | 8 |
| 32.09 | 2.71 | 19 |
| 34.91 | 2.57 | 15 |

Preparation Example I-2

4g deionized water, 0.75g 4-pyrrolidinylpyridine, 0.42g aluminum isopropoxide, 1.5g silica sol, and 0.5g hydrofluoric acid were mixed uniformly to obtain a component with a composition of $1SiO_2:0.1Al_2O_3:1F^-:29H_2O:0.5OSDA$, followed by open heating pretreatment at 80 °C, and a block mixture was obtained. After the raw material mixture was heated and pretreated, the molar ratio of silicon source (calculated as $SiO_2$) and water during crystallization was 1:5, and then crystallized in a crystallizing kettle at 170 °C for 15 days. After taking out the product, it was washed three times with deionized water, dried and calcined at 550 °C for 6 hours to obtain the required H-type silica-alumina molecular sieve (with ATS structure).

2.5 mL of 3.2 g/L ruthenium chloride solution was added dropwise onto 1 g of the H-type silica-alumina molecular sieve. After drying at 80 °C for 2 hours, it was reduced in a fixed-bed reactor for 3 hours at 350°C and a hydrogen flow rate of 10 mL/min to obtain the required catalyst.

The XRD spectrum data of the catalyst are shown in Table I-2, the XRD spectrum is similar to Figure 1. In the catalyst composition, $n(SiO_2): n(Al_2O_3)=10$, the Ru mass fraction is 0.6%, and the chemical composition of the catalyst is expressed as "$xM \cdot ySiO_2 \cdot zAl_2O_3$", then x/y=0.0041,y/z=10. The properties of the resulting catalyst are listed in Table I-13.

The SEM of the catalyst is similar to Figure 3. In the catalyst, the crystals have a long strip shape, the length of the crystals is 0.4-1.5 $\mu$m, and the aspect ratio is 2-10.

Table I-2 XRD spectrum data of the catalyst obtained in Preparation Example I-2

| $2\theta$ (°) | d-spacing (Å) | Relative intensity ($I/I_0 \times 100$) |
|---|---|---|
| 7.89 | 11.20 | 100 |
| 16.52 | 5.37 | 36 |
| 19.18 | 4.62 | 58 |
| 20.79 | 4.27 | 36 |
| 21.89 | 4.04 | 23 |
| 22.41 | 3.97 | 21 |
| 27.28 | 3.27 | 21 |
| 28.52 | 3.13 | 9 |
| 29.28 | 3.05 | 12 |
| 30.56 | 2.92 | 6 |
| 32.09 | 2.71 | 17 |
| 34.91 | 2.57 | 15 |

Preparation Example I-3

4g deionized water, 0.75g 4-pyrrolidinylpyridine, 0.42g aluminum isopropoxide, 1.5g silica sol, and 0.5g hydrofluoric acid were mixed uniformly to obtain a component with a composition of $1SiO_2:0.1Al_2O_3:1F^-:29H_2O:0.5OSDA$, followed by open heating pretreatment at 80 °C, and a block mixture was obtained. After the raw material mixture was heated and pretreated, the molar ratio of silicon source (calculated as $SiO_2$) and water during crystallization was 1:5, and then crystallized in a crystallizing kettle at 170 °C for 15 days. After taking out the product, it was washed three times with deionized water, dried and calcined at 550 °C for 6 hours to obtain the required H-type silica-alumina molecular sieve (with ATS structure).

6 mL of 3.2 g/L ruthenium chloride solution was added dropwise onto 1 g of the H-type silica-alumina molecular sieve. After drying at 80 °C for 2 hours, it was reduced in a fixed-bed reactor for 3 hours at 350°C and a hydrogen flow rate of 10 mL/min to obtain the required catalyst.

The XRD spectrum data of the catalyst are shown in Table I-3, the XRD spectrum is similar to Figure 1. In the catalyst composition, n($SiO_2$): n($Al_2O_3$)=10, the Ru mass fraction is 1.5%, and the chemical composition of the catalyst is expressed as "$xM \cdot ySiO_2 \cdot zAl_2O_3$", then x/y=0.0104, y/z=10. The properties of the resulting catalyst are listed in Table I-13.

The SEM of the catalyst is similar to Figure 3. In the catalyst, the crystals have a long strip shape, the length of the crystals is 0.4-1.5 $\mu$m, and the aspect ratio is 2-10.

Table I-3 XRD spectrum data of the catalyst obtained in Preparation Example I-3

| $2\theta$ (°) | d-spacing (Å) | Relative intensity ($I/I_0 \times 100$) |
|---|---|---|
| 7.89 | 11.20 | 100 |
| 16.59 | 5.33 | 42 |
| 19.19 | 4.62 | 51 |
| 20.80 | 4.27 | 34 |
| 21.86 | 4.06 | 15 |
| 22.40 | 3.97 | 21 |

(continued)

| 2θ(°) | d-spacing (Å) | Relative intensity (I/I$_0$ × 100) |
|---|---|---|
| 27.28 | 3.27 | 19 |
| 28.50 | 3.14 | 11 |
| 29.28 | 3.05 | 12 |
| 30.56 | 2.92 | 8 |
| 32.10 | 2.71 | 19 |
| 34.91 | 2.57 | 15 |

Preparation Example I-4

4g deionized water, 1.1g 4-pyrrolidinylpyridine, 0.42g aluminum isopropoxide, 1.5g silica sol, and 0.75g hydrofluoric acid were mixed uniformly to obtain a component with a composition of $1SiO_2:0.1Al_2O_3:1.5F^-:29H_2O:0.75OSDA$, followed by open heating pretreatment at 80 °C, and a block mixture was obtained. After the raw material mixture was heated and pretreated, the molar ratio of silicon source (calculated as $SiO_2$) and water during crystallization was 1:4.5, and then crystallized in a crystallizing kettle at 170 °C for 15 days. After taking out the product, it was washed three times with deionized water, dried and calcined at 550 °C for 6 hours to obtain the required H-type silica-alumina molecular sieve (with ATS structure).

1.2 mL of 3.2 g/L ruthenium chloride solution was added dropwise onto 1 g of the H-type silica-alumina molecular sieve. After drying at 80 °C for 2 hours, it was reduced in a fixed-bed reactor for 3 hours at 350°C and a hydrogen flow rate of 10 mL/min to obtain the required catalyst.

The XRD spectrum data of the catalyst are shown in Table I-4, the XRD spectrum is similar to Figure 1. In the catalyst composition, $n(SiO_2): n(Al_2O_3)=10$, the Ru mass fraction is 0.3%, and the chemical composition of the catalyst is expressed as "$xM \cdot ySiO_2 \cdot zAl_2O_3$", then x/y=0.0021, y/z=10. The properties of the resulting catalyst are listed in Table I-13.

The SEM of the catalyst is similar to Figure 3. In the catalyst, the crystals have a long strip shape, the length of the crystals is 0.4-2.0 μm, and the aspect ratio is 2-15.

Table I-4 XRD spectrum data of the catalyst obtained in Preparation Example I-4

| 2θ(°) | d-spacing (Å) | Relative intensity (I/I$_0$ × 100) |
|---|---|---|
| 7.89 | 11.20 | 100 |
| 16.51 | 5.37 | 46 |
| 19.18 | 4.62 | 56 |
| 20.79 | 4.27 | 43 |
| 21.89 | 4.04 | 15 |
| 22.40 | 3.97 | 23 |
| 27.28 | 3.27 | 21 |
| 28.52 | 3.13 | 14 |
| 29.21 | 3.07 | 13 |
| 30.56 | 2.92 | 9 |
| 32.10 | 2.71 | 19 |
| 34.91 | 2.57 | 15 |

Preparation Example I-5

4g deionized water, 1.5g 4-pyrrolidinylpyridine, 0.42g aluminum isopropoxide, 1.5g silica sol, and 1g hydrofluoric acid were mixed uniformly to obtain a component with a composition of $1SiO_2:0.1Al_2O_3:2F^-:30H_2O:1OSDA$, followed by open heating pretreatment at 80 °C, and a block mixture was obtained. After the raw material mixture was heated and pretreated, the molar ratio of silicon source (calculated as $SiO_2$) and water during crystallization was 1:4.5, and then crystallized in a crystallizing kettle at 170 °C for 15 days. After taking out the product, it was washed three times with deionized water, dried and calcined at 550 °C for 6 hours to obtain the required H-type silica-alumina molecular sieve (with ATS structure).

1.2 mL of 3.2 g/L ruthenium chloride solution was added dropwise onto 1 g of the H-type silica-alumina molecular sieve. After drying at 80 °C for 2 hours, it was reduced in a fixed-bed reactor for 3 hours at 350°C and a hydrogen flow rate of 10 mL/min to obtain the required catalyst.

The XRD spectrum data of the catalyst are shown in Table I-5, the XRD spectrum is similar to Figure 1. In the catalyst composition, $n(SiO_2): n(Al_2O_3)=10$, the Ru mass fraction is 0.3%, and the chemical composition of the catalyst is expressed as "$xM \cdot ySiO_2 \cdot zAl_2O_3$", then x/y=0.0021, y/z=10. The properties of the resulting catalyst are listed in Table I-13.

The SEM of the catalyst is similar to Figure 3. In the catalyst, the crystals have a long strip shape, the length of the crystals is 0.5-2.5 $\mu$m, and the aspect ratio is 3-20.

Table I-5 XRD spectrum data of the catalyst obtained in Preparation Example I-5

| $2\theta(°)$ | d-spacing (Å) | Relative intensity ($I/I_0 \times 100$) |
| --- | --- | --- |
| 7.89 | 11.20 | 100 |
| 16.51 | 5.37 | 44 |
| 19.20 | 4.62 | 53 |
| 20.77 | 4.27 | 43 |
| 21.89 | 4.04 | 18 |
| 22.40 | 3.97 | 23 |
| 27.25 | 3.27 | 27 |
| 28.50 | 3.12 | 14 |
| 29.21 | 3.07 | 13 |
| 30.56 | 2.92 | 11 |
| 32.14 | 2.70 | 18 |
| 34.91 | 2.57 | 13 |

Preparation Example I-6

4g deionized water, 0.75g 4-pyrrolidinylpyridine, 0.42g aluminum isopropoxide, 1.5g silica sol, and 0.5g hydrofluoric acid were mixed uniformly to obtain a component with a composition of $1SiO_2:0.1Al_2O_3:1F^-:29H_2O:0.5OSDA$, followed by open heating pretreatment at 80 °C, and a block mixture was obtained. After the raw material mixture was heated and pretreated, the molar ratio of silicon source (calculated as $SiO_2$) and water during crystallization was 1:5, and then crystallized in a crystallizing kettle at 170 °C for 15 days. After taking out the product, it was washed three times with deionized water, dried and calcined at 550 °C for 6 hours to obtain the required H-type silica-alumina molecular sieve (with ATS structure).

1.2 mL of 2.7 g/L palladium chloride solution was added dropwise onto 1 g of the H-type silica-alumina molecular sieve. After drying at 80 °C for 2 hours, it was reduced in a fixed-bed reactor for 3 hours at 350°C and a hydrogen flow rate of 10 mL/min to obtain the required catalyst.

The XRD spectrum data of the catalyst are shown in Table I-6, the XRD spectrum is similar to Figure 1. In the catalyst composition, $n(SiO_2): n(Al_2O_3)=10$, the Pd mass fraction is 0.3%, and the chemical composition of the catalyst is expressed as "$xM \cdot ySiO_2 \cdot zAl_2O_3$", then x/y=0.0021, y/z=10. The properties of the resulting catalyst are listed in Table I-13.

The SEM of the catalyst is similar to Figure 3. In the catalyst, the crystals have a long strip shape, the length of the crystals is 0.4-1.5 $\mu$m, and the aspect ratio is 2-10.

Table I-6 XRD spectrum data of the catalyst obtained in Preparation Example I-6

| $2\theta(°)$ | d-spacing (Å) | Relative intensity ($I/I_0 \times 100$) |
|---|---|---|
| 7.89 | 11.20 | 100 |
| 16.60 | 5.33 | 44 |
| 19.15 | 4.63 | 48 |
| 20.79 | 4.27 | 41 |
| 21.88 | 4.05 | 17 |
| 22.40 | 3.97 | 20 |
| 27.28 | 3.27 | 20 |
| 28.52 | 3.13 | 7 |
| 29.28 | 3.05 | 16 |
| 30.56 | 2.92 | 8 |
| 32.08 | 2.73 | 17 |
| 34.91 | 2.57 | 14 |

Preparation Example I-7

4g deionized water, 0.75g 4-pyrrolidinylpyridine, 0.21g aluminum isopropoxide, 1.5g silica sol, and 0.5g hydrofluoric acid were mixed uniformly to obtain a component with a composition of $1SiO_2:0.05Al_2O_3:1F^-:29H_2O:0.5OSDA$, followed by open heating pretreatment at 80 °C, and a block mixture was obtained. After the raw material mixture was heated and pretreated, the molar ratio of silicon source (calculated as $SiO_2$) and water during crystallization was 1:5, and then crystallized in a crystallizing kettle at 170 °C for 15 days. After taking out the product, it was washed three times with deionized water, dried and calcined at 550 °C for 6 hours to obtain the required H-type silica-alumina molecular sieve (with ATS structure).

1.2 mL of 3.2 g/L ruthenium chloride solution was added dropwise onto 1 g of the H-type silica-alumina molecular sieve. After drying at 80 °C for 2 hours, it was reduced in a fixed-bed reactor for 3 hours at 350°C and a hydrogen flow rate of 10 mL/min to obtain the required catalyst.

The XRD spectrum data of the catalyst are shown in Table I-7, the XRD spectrum is similar to Figure 1. In the catalyst composition, $n(SiO_2): n(Al_2O_3)=20$, the Ru mass fraction is 0.3%, and the chemical composition of the catalyst is expressed as "$xM \cdot ySiO_2 \cdot zAl_2O_3$", then x/y=0.0021, y/z=20. The properties of the resulting catalyst are listed in Table I-13.

The SEM of the catalyst is similar to Figure 3. In the catalyst, the crystals have a long strip shape, the length of the crystals is 0.4-1.5 $\mu$m, and the aspect ratio is 2-12.

Table I-7 XRD spectrum data of the catalyst obtained in Preparation Example I-7

| $2\theta(°)$ | d-spacing (Å) | Relative intensity ($I/I_0 \times 100$) |
|---|---|---|
| 7.86 | 11.21 | 100 |
| 16.55 | 5.35 | 49 |
| 19.19 | 4.62 | 66 |
| 20.76 | 4.27 | 46 |
| 21.86 | 4.06 | 20 |
| 22.42 | 3.96 | 36 |
| 27.28 | 3.27 | 30 |
| 28.50 | 3.14 | 13 |

(continued)

| 2θ(°) | d-spacing (Å) | Relative intensity (I/I$_0$ × 100) |
|---|---|---|
| 29.27 | 3.05 | 16 |
| 30.56 | 2.92 | 10 |
| 34.90 | 2.57 | 17 |

Preparation Example I-8

4g deionized water, 0.75g 4-pyrrolidinylpyridine, 0.08g aluminum isopropoxide, 1.5g silica sol, and 0.5g hydrofluoric acid were mixed uniformly to obtain a component with a composition of $1SiO_2:0.02Al_2O_3:1F^-:29H_2O:0.5OSDA$, followed by open heating pretreatment at 80 °C, and a block mixture was obtained. After the raw material mixture was heated and pretreated, the molar ratio of silicon source (calculated as $SiO_2$) and water during crystallization was 1:5, and then crystallized in a crystallizing kettle at 170 °C for 15 days. After taking out the product, it was washed three times with deionized water, dried and calcined at 550 °C for 6 hours to obtain the required H-type silica-alumina molecular sieve (with ATS structure).

1.2 mL of 3.2 g/L ruthenium chloride solution was added dropwise onto 1 g of the H-type silica-alumina molecular sieve. After drying at 80 °C for 2 hours, it was reduced in a fixed-bed reactor for 3 hours at 350°C and a hydrogen flow rate of 10 mL/min to obtain the required catalyst.

The XRD spectrum data of the catalyst are shown in Table I-8, the XRD spectrum is similar to Figure 1. In the catalyst composition, n($SiO_2$): n($Al_2O_3$)=50, the Ru mass fraction is 0.3%, and the chemical composition of the catalyst is expressed as "$xM \cdot ySiO_2 \cdot zAl_2O_3$", then x/y=0.0021, y/z=50. The properties of the resulting catalyst are listed in Table I-13.

The SEM of the catalyst is similar to Figure 3. In the catalyst, the crystals have a long strip shape, the length of the crystals is 0.4-1.5 μm, and the aspect ratio is 2-12.

Table I-8 XRD spectrum data of the catalyst obtained in Preparation Example I-8

| 2θ(°) | d-spacing (Å) | Relative intensity (I/I$_0$ × 100) |
|---|---|---|
| 7.86 | 11.21 | 100 |
| 16.55 | 5.35 | 41 |
| 19.22 | 4.61 | 43 |
| 20.80 | 4.27 | 32 |
| 21.88 | 4.05 | 14 |
| 22.42 | 3.96 | 17 |
| 27.28 | 3.27 | 16 |
| 28.47 | 3.14 | 15 |
| 29.27 | 3.05 | 12 |
| 30.55 | 2.92 | 7 |
| 32.08 | 2.73 | 22 |
| 34.90 | 2.58 | 11 |

Preparation Example I-9

4g deionized water, 0.75g 4-pyrrolidinylpyridine, 0.42g aluminum isopropoxide, 1.5g silica sol, and 0.5g hydrofluoric acid were mixed uniformly to obtain a component with a composition of $1SiO_2:0.1Al_2O_3:1F^-:29H_2O:0.5OSDA$, followed by open heating pretreatment at 80 °C, and a block mixture was obtained. After the raw material mixture was heated and pretreated, the molar ratio of silicon source (calculated as $SiO_2$) and water during crystallization was 1:5, and then crystallized in a crystallizing kettle at 170 °C for 15 days. After taking out the product, it was washed three times with deionized water, dried and calcined at 550 °C for 6 hours to obtain the required H-type silica-alumina molecular sieve (with ATS structure).

1.2 mL of 3.2 g/L ruthenium chloride solution was added dropwise onto 1 g of the H-type silica-alumina molecular sieve. After drying at 80 °C for 2 hours, it was reduced in a fixed-bed reactor for 3 hours at 450°C and a hydrogen flow rate of 10 mL/min to obtain a catalyst precursor. 0.3g dimethyldimethoxysilane, 1g catalyst precursor and 20mL ethanol solvent were mixed, refluxed at 75°C for 24h, centrifuged with water, washed, and dried at 80°C for 12h to obtain the catalyst. The properties of the resulting catalyst are listed in Table I-13.

The XRD spectrum data of the catalyst are shown in Table I-9, the XRD spectrum is similar to Figure 1. The SEM of the catalyst is similar to Figure 3. In the catalyst, the crystals have a long strip shape, the length of the crystals is 0.4-1.5 $\mu$m, and the aspect ratio is 2-10.

Table I-9 XRD spectrum data of the catalyst obtained in Preparation Example I-9

| $2\theta(°)$ | d-spacing (Å) | Relative intensity (I/I$_0$ $\times$ 100) |
| --- | --- | --- |
| 7.89 | 11.20 | 100 |
| 16.52 | 5.37 | 37 |
| 19.18 | 4.62 | 55 |
| 20.79 | 4.27 | 33 |
| 21.89 | 4.04 | 21 |
| 22.41 | 3.97 | 20 |
| 27.28 | 3.27 | 20 |
| 28.52 | 3.13 | 9 |
| 29.28 | 3.05 | 11 |
| 30.56 | 2.92 | 6 |
| 32.09 | 2.71 | 15 |
| 34.91 | 2.57 | 14 |

Preparation Example I-10

4g deionized water, 1.5g 4-pyrrolidinylpyridine, 0.42g aluminum isopropoxide, 1.5g silica sol, and 1g hydrofluoric acid were mixed uniformly to obtain a component with a composition of $1SiO_2:0.1AlO_3:2F^-:30H_2O:1OSDA$, followed by open heating pretreatment at 80 °C, and a block mixture was obtained. After the raw material mixture was heated and pretreated, the molar ratio of silicon source (calculated as $SiO_2$) and water during crystallization was 1:4.5, and then crystallized in a crystallizing kettle at 170 °C for 15 days. After taking out the product, it was washed three times with deionized water, dried and calcined at 550 °C for 6 hours to obtain the required H-type silica-alumina molecular sieve (with ATS structure).

2.5 mL of 3.2 g/L ruthenium chloride solution was added dropwise onto 1 g of the H-type silica-alumina molecular sieve. After drying at 80 °C for 2 hours, it was reduced in a fixed-bed reactor for 3 hours at 450°C and a hydrogen flow rate of 10 mL/min to obtain a catalyst precursor. 0.3g phenyltrimethoxysilane, 1g catalyst precursor and 20mL ethanol solvent were mixed, refluxed at 75°C for 24h, centrifuged with water, washed, and dried at 80°C for 12h to obtain the catalyst. The properties of the resulting catalyst are listed in Table I-13.

The XRD spectrum data of the catalyst are shown in Table I-10, the XRD spectrum is similar to Figure 1. The SEM of the catalyst is similar to Figure 3. In the catalyst, the crystals have a long strip shape, the length of the crystals is 0.5-2.5 $\mu$m, and the aspect ratio is 3-20.

Table I-10 XRD spectrum data of the catalyst obtained in Preparation Example I-10

| $2\theta(°)$ | d-spacing (Å) | Relative intensity (I/I$_0$ $\times$ 100) |
| --- | --- | --- |
| 7.89 | 11.20 | 100 |
| 16.51 | 5.37 | 44 |

(continued)

| 2θ(°) | d-spacing (Å) | Relative intensity (I/I$_0$ × 100) |
|---|---|---|
| 19.20 | 4.62 | 53 |
| 20.77 | 4.27 | 43 |
| 21.89 | 4.04 | 18 |
| 22.40 | 3.97 | 23 |
| 27.25 | 3.27 | 27 |
| 28.50 | 3.12 | 14 |
| 29.21 | 3.07 | 13 |
| 30.56 | 2.92 | 11 |
| 32.14 | 2.70 | 18 |
| 34.91 | 2.57 | 13 |

Preparation Example I-11

4g deionized water, 0.75g 4-pyrrolidinylpyridine, 0.42g aluminum isopropoxide, 1.5g silica sol, and 0.5g hydrofluoric acid were mixed uniformly to obtain a component with a composition of 1SiO$_2$:0.1Al$_2$O$_3$:1F$^-$:29H$_2$O:0.5OSDA, followed by open heating pretreatment at 80 °C, and a block mixture was obtained. After the raw material mixture was heated and pretreated, the molar ratio of silicon source (calculated as SiO$_2$) and water during crystallization was 1:5, and then crystallized in a crystallizing kettle at 170 °C for 15 days. After taking out the product, it was washed three times with deionized water, dried and calcined at 550 °C for 6 hours to obtain the required H-type silica-alumina molecular sieve (with ATS structure).

1.2 mL of 2.7 g/L palladium chloride solution was added dropwise onto 1 g of the H-type silica-alumina molecular sieve. After drying at 80 °C for 2 hours, it was reduced in a fixed-bed reactor for 3 hours at 450°C and a hydrogen flow rate of 10 mL/min to obtain a catalyst precursor. 0.3g phenyltrimethoxysilane, 1g catalyst precursor and 20mL ethanol solvent were mixed, refluxed at 75°C for 24h, centrifuged with water, washed, and dried at 80°C for 12h to obtain the catalyst. The properties of the resulting catalyst are listed in Table I-13.

The XRD spectrum data of the catalyst are shown in Table I-11, the XRD spectrum is similar to Figure 1. The SEM of the catalyst is similar to Figure 3. In the catalyst, the crystals have a long strip shape, the length of the crystals is 0.4-1.5 μm, and the aspect ratio is 2-10.

Table I-11 XRD spectrum data of the catalyst obtained in Preparation Example I-11

| 2θ(°) | d-spacing (Å) | Relative intensity (I/I$_0$ × 100) |
|---|---|---|
| 7.89 | 11.20 | 100 |
| 16.60 | 5.33 | 44 |
| 19.15 | 4.63 | 48 |
| 20.79 | 4.27 | 41 |
| 21.88 | 4.05 | 17 |
| 22.40 | 3.97 | 20 |
| 27.28 | 3.27 | 20 |
| 28.52 | 3.13 | 7 |
| 29.28 | 3.05 | 16 |
| 30.56 | 2.92 | 8 |
| 32.08 | 2.73 | 17 |
| 34.91 | 2.57 | 14 |

Preparation Example I-12

4g deionized water, 0.75g 4-pyrrolidinylpyridine, 0.21g aluminum isopropoxide, 1.5g silica sol, and 0.5g hydrofluoric acid were mixed uniformly to obtain a component with a composition of $1SiO_2:0.05Al_2O_3:1F^-:29H_2O:0.5OSDA$, followed by open heating pretreatment at 80 °C, and a block mixture was obtained. After the raw material mixture was heated and pretreated, the molar ratio of silicon source (calculated as $SiO_2$) and water during crystallization was 1:5, and then crystallized in a crystallizing kettle at 170 °C for 15 days. After taking out the product, it was washed three times with deionized water, dried and calcined at 550 °C for 6 hours to obtain the required H-type silica-alumina molecular sieve (with ATS structure).

1.2 mL of 3.2 g/L ruthenium chloride solution was added dropwise onto 1 g of the H-type silica-alumina molecular sieve. After drying at 80 °C for 2 hours, it was reduced in a fixed-bed reactor for 3 hours at 450°C and a hydrogen flow rate of 10 mL/min to obtain a catalyst precursor. 0.3g phenyltrimethoxysilane, 1g catalyst precursor and 20mL ethanol solvent were mixed, refluxed at 75°C for 24h, centrifuged with water, washed, and dried at 80°C for 12h to obtain the catalyst. The properties of the resulting catalyst are listed in Table I-13.

The XRD spectrum data of the catalyst are shown in Table I-12, the XRD spectrum is similar to Figure 1. The SEM of the catalyst is similar to Figure 3. In the catalyst, the crystals have a long strip shape, the length of the crystals is 0.4-1.5 $\mu$m, and the aspect ratio is 2-12.

Table I-12 XRD spectrum data of the catalyst obtained in Preparation Example I-12

| $2\theta(°)$ | d-spacing (Å) | Relative intensity ($I/I_0 \times 100$) |
|---|---|---|
| 7.86 | 11.21 | 100 |
| 16.55 | 5.35 | 49 |
| 19.19 | 4.62 | 66 |
| 20.76 | 4.27 | 46 |
| 21.86 | 4.06 | 20 |
| 22.42 | 3.96 | 36 |
| 27.28 | 3.27 | 30 |
| 28.50 | 3.14 | 13 |
| 29.27 | 3.05 | 16 |
| 30.56 | 2.92 | 10 |
| 34.90 | 2.57 | 17 |

Example I-1

0.25g of the catalyst synthesized in Preparation Example I-1 was added to the high-pressure reactor, and then 8g of benzene was added to the reactor, and hydrogen was charged to bring the system pressure to 1.2MPa. Then the temperature of the system was raised to 150 °C, and the reaction was completed after 4 hours. Evaluation data are summarized in Table I-14.

Example I-2

0.25g of the catalyst synthesized in Preparation Example I-1 was added to the high-pressure reactor, and then 8g of benzene was added to the reactor, and hydrogen was charged to bring the system pressure to 1.6MPa. Then the temperature of the system was raised to 150 °C, and the reaction was completed after 4 hours. Evaluation data are summarized in Table I-14.

Example I-3

0.25g of the catalyst synthesized in Preparation Example I-1 was added to the high-pressure reactor, and then 8g of benzene was added to the reactor, and hydrogen was charged to bring the system pressure to 2.0MPa. Then the temperature of the system was raised to 150 °C, and the reaction was completed after 4 hours. Evaluation data are summarized in Table I-14.

Example I-4

0.25g of the catalyst synthesized in Preparation Example I-1 was added to the high-pressure reactor, and then 8g of benzene was added to the reactor, and hydrogen was charged to bring the system pressure to 1.2MPa. Then the temperature of the system was raised to 180 °C, and the reaction was completed after 4 hours. Evaluation data are summarized in Table I-14.

Example I-5

0.25g of the catalyst synthesized in Preparation Example I-1 was added to the high-pressure reactor, and then 8g of benzene was added to the reactor, and hydrogen was charged to bring the system pressure to 1.2MPa. Then the temperature of the system was raised to 200 °C, and the reaction was completed after 4 hours. Evaluation data are summarized in Table I-14.

Example I-6

0.25g of the catalyst synthesized in Preparation Example I-2 was added to the high-pressure reactor, and then 8g of benzene was added to the reactor, and hydrogen was charged to bring the system pressure to 1.2MPa. Then the temperature of the system was raised to 150 °C, and the reaction was completed after 4 hours. Evaluation data are summarized in Table I-14.

Example I-7

0.25g of the catalyst synthesized in Preparation Example I-3 was added to the high-pressure reactor, and then 8g of benzene was added to the reactor, and hydrogen was charged to bring the system pressure to 1.2MPa. Then the temperature of the system was raised to 150 °C, and the reaction was completed after 4 hours. Evaluation data are summarized in Table I-14.

Example I-8

0.25g of the catalyst synthesized in Preparation Example I-4 was added to the high-pressure reactor, and then 8g of benzene was added to the reactor, and hydrogen was charged to bring the system pressure to 1.2MPa. Then the temperature of the system was raised to 150 °C, and the reaction was completed after 4 hours. Evaluation data are summarized in Table I-14.

Example I-9

0.25g of the catalyst synthesized in Preparation Example I-5 was added to the high-pressure reactor, and then 8g of benzene was added to the reactor, and hydrogen was charged to bring the system pressure to 1.2MPa. Then the temperature of the system was raised to 150 °C, and the reaction was completed after 4 hours. Evaluation data are summarized in Table I-14.

Example I-10

0.25g of the catalyst synthesized in Preparation Example I-6 was added to the high-pressure reactor, and then 8g of benzene was added to the reactor, and hydrogen was charged to bring the system pressure to 1.2MPa. Then the temperature of the system was raised to 150 °C, and the reaction was completed after 4 hours. Evaluation data are summarized in Table I-14.

Example 1-11

0.25g of the catalyst synthesized in Preparation Example I-7 was added to the high-pressure reactor, and then 8g of benzene was added to the reactor, and hydrogen was charged to bring the system pressure to 1.2MPa. Then the temperature of the system was raised to 150 °C, and the reaction was completed after 4 hours. Evaluation data are summarized in Table I-14.

Example I-12

0.25g of the catalyst synthesized in Preparation Example I-9 was added to the high-pressure reactor, and then 8g of benzene was added to the reactor, and hydrogen was charged to bring the system pressure to 1.2MPa. Then the temperature of the system was raised to 150 °C, and the reaction was completed after 4 hours. Evaluation data are summarized in Table I-14.

Example I-13

0.25g of the catalyst synthesized in Preparation Example I-9 was added to the high-pressure reactor, and then 8g of benzene was added to the reactor, and hydrogen was charged to bring the system pressure to 1.6MPa. Then the temperature of the system was raised to 150 °C, and the reaction was completed after 4 hours. Evaluation data are summarized in Table I-14.

Example I-14

0.25g of the catalyst synthesized in Preparation Example I-9 was added to the high-pressure reactor, and then 8g of benzene was added to the reactor, and hydrogen was charged to bring the system pressure to 2.0MPa. Then the temperature of the system was raised to 150 °C, and the reaction was completed after 4 hours. Evaluation data are summarized in Table I-14.

Example I-15

0.25g of the catalyst synthesized in Preparation Example I-9 was added to the high-pressure reactor, and then 8g of benzene was added to the reactor, and hydrogen was charged to bring the system pressure to 1.2MPa. Then the temperature of the system was raised to 180 °C, and the reaction was completed after 4 hours. Evaluation data are summarized in Table I-14.

Example I-16

0.25g of the catalyst synthesized in Preparation Example I-9 was added to the high-pressure reactor, and then 8g of benzene was added to the reactor, and hydrogen was charged to bring the system pressure to 1.2MPa. Then the temperature of the system was raised to 200 °C, and the reaction was completed after 4 hours. Evaluation data are summarized in Table I-14.

Example I-17

0.25g of the catalyst synthesized in Preparation Example I-10 was added to the high-pressure reactor, and then 8g of benzene was added to the reactor, and hydrogen was charged to bring the system pressure to 1.2MPa. Then the temperature of the system was raised to 150 °C, and the reaction was completed after 4 hours. Evaluation data are summarized in Table I-14.

Example I-18

0.25g of the catalyst synthesized in Preparation Example I-11 was added to the high-pressure reactor, and then 8g of benzene was added to the reactor, and hydrogen was charged to bring the system pressure to 1.2MPa. Then the temperature of the system was raised to 150 °C, and the reaction was completed after 4 hours. Evaluation data are summarized in Table I-14.

Example I-19

0.25g of the catalyst synthesized in Preparation Example I-12 was added to the high-pressure reactor, and then 8g of benzene was added to the reactor, and hydrogen was charged to bring the system pressure to 1.2MPa. Then the temperature of the system was raised to 150 °C, and the reaction was completed after 4 hours. Evaluation data are summarized in Table I-14.

Table I-13 Physicochemical properties of the catalysts obtained in the Preparation Examples

| Example number | Particle size of metal M particles (nm) | Metal M and content thereof (wt%) | Content of the metal M on the external surface to the external surface elements (wt%) | Distribution coefficient of the metal M on the external surface (%) | Hydrocarbyl modifying component and content thereof (wt%) | n (Si): n (Al) |
|---|---|---|---|---|---|---|
| Preparation Example I-1 | 0.5-2 | 0.30 (ruthenium) | 0.32 | 7.9 | 0 | 5 |
| Preparation Example I-2 | 0.5-2 | 0.59 (ruthenium) | 0.60 | 7.2 | 0 | 5 |
| Preparation Example I-3 | 0.5-2 | 1.5 (ruthenium) | 1.5 | 7.2 | 0 | 5 |
| Preparation Example I-4 | 0.5-2 | 0.29 (ruthenium) | 0.29 | 7.3 | 0 | 5 |
| Preparation Example I-5 | 0.5-2 | 0.29 (ruthenium) | 0.29 | 7.3 | 0 | 5 |
| Preparation Example I-6 | 0.5-2 | 0.27 (palladium) | 0.25 | 6.8 | 0 | 5 |
| Preparation Example I-7 | 0.5-2 | 0.30 (ruthenium) | 0.32 | 7.6 | 0 | 10 |
| Preparation Example I-8 | 0.5-2 | 0.31 (ruthenium) | 0.31 | 7.8 | 0 | 25 |
| Preparation Example I-9 | 0.5-2 | 0.29 (ruthenium) | 0.11 | 2.3 | 5.9 (methyl) | 5 |
| Preparation Example I-10 | 0.5-2 | 0.61 (ruthenium) | 0.22 | 2.2 | 11.4 (phenyl) | 5 |
| Preparation Example I-11 | 0.5-2 | 0.28 (palladium) | 0.07 | 1.6 | 11.6 (phenyl) | 5 |
| Preparation Example I-12 | 0.5-2 | 0.30 (ruthenium) | 0.08 | 1.7 | 11.5 (phenyl) | 10 |

Table I-13 (continued) Physicochemical properties of the catalysts obtained in the Preparation Examples

| Example number | Specific surface area ($m^2/g$) | External specific surface area ($m^2/g$) | Micropore volume ($cm^3/g$) | $C_{B, Py} / C_{L, Py}$ | Total acid content ($\mu mol/g$) | Relative acid equivalent of the external surface (%) |
|---|---|---|---|---|---|---|
| Preparation Example I-1 | 433 | 32 | 0.16 | 5.6 | 1271 | 100 |

(continued)

| Example number | Specific surface area (m²/g) | External specific surface area (m²/g) | Micropore volume (cm³/g) | $C_{B, Py}/C_{L, Py}$ | Total acid content (μmol/g) | Relative acid equivalent of the external surface (%) |
|---|---|---|---|---|---|---|
| Preparation Example I-2 | 437 | 31 | 0.16 | 5.6 | 1278 | 100 |
| Preparation Example I-3 | 429 | 31 | 0.15 | 5.6 | 1275 | 100 |
| Preparation Example I-4 | 454 | 33 | 0.17 | 5.6 | 1269 | 100 |
| Preparation Example I-5 | 466 | 34 | 0.16 | 5.7 | 1272 | 100 |
| Preparation Example I-6 | 422 | 31 | 0.16 | 5.6 | 1285 | 100 |
| Preparation Example I-7 | 379 | 27 | 0.14 | 5.9 | 1249 | 100 |
| Preparation Example I-8 | 321 | 25 | 0.11 | 6.1 | 1016 | 100 |
| Preparation Example I-9 | 441 | 29 | 0.15 | 5.6 | 1262 | 34 |
| Preparation Example I-10 | 438 | 27 | 0.15 | 5.7 | 1251 | 27 |
| Preparation Example I-11 | 436 | 27 | 0.14 | 5.6 | 1271 | 25 |
| Preparation Example I-12 | 441 | 33 | 0.15 | 5.6 | 1224 | 26 |

Table I-14 Catalytic evaluation conditions and results of the Examples

| Example number | H₂ pressure (MPa) | Reaction temperature (°C) | Yield of cyclohexylbenzene (%) | Selectivity of cyclohexylbenzene (%) |
|---|---|---|---|---|
| Example I-1 | 1.2 | 150 | 59 | 93.7 |
| Example I-2 | 1.6 | 150 | 63 | 88.7 |
| Example I-3 | 2.0 | 150 | 65 | 85.4 |
| Example I-4 | 1.2 | 180 | 59 | 93.6 |
| Example I-5 | 1.2 | 200 | 59 | 93.1 |
| Example I-6 | 1.2 | 150 | 59 | 93.4 |
| Example I-7 | 1.2 | 150 | 51 | 91.9 |
| Example I-8 | 1.2 | 150 | 59 | 93.2 |
| Example I-9 | 1.2 | 150 | 58 | 92.9 |
| Example I-10 | 1.2 | 150 | 59 | 93.6 |
| Example I-11 | 1.2 | 150 | 58 | 92.7 |
| Example I-12 | 1.2 | 150 | 60 | 94.7 |
| Example I-13 | 1.6 | 150 | 64 | 90.8 |
| Example I-14 | 2.0 | 150 | 66 | 90.5 |
| Example I-15 | 1.2 | 180 | 60 | 95.4 |
| Example I-16 | 1.2 | 200 | 59 | 94.5 |
| Example I-17 | 1.2 | 150 | 59 | 95.6 |
| Example I-18 | 1.2 | 150 | 59 | 95.8 |
| Example I-19 | 1.2 | 150 | 58 | 94.4 |

Comparative Example I-1

1. Catalyst preparation:

The catalyst was prepared with reference to Preparation Example I-1, except that the H-type silica-alumina molecular sieve was changed to an H-type Y molecular sieve (with FAU structure) with n (Si): n (Al) = 10.
1.2 mL of 3.2 g/L ruthenium chloride solution was added dropwise onto 1 g of the above molecular sieve. After drying at 80 °C for 2 hours, it was reduced in a fixed-bed reactor for 3 hours at 350 °C and a hydrogen flow rate of 10 mL/min to obtain the required catalyst.

2. Catalyst evaluation:

The catalyst evaluation method is shown in Example I-1. The composition and evaluation results of the catalyst are listed in Table I-15.

Comparative Example I-2

1. Catalyst preparation:

The catalyst was prepared with reference to Preparation Example I-1, except that the H-type silica-alumina molecular sieve was changed to an H-type Y molecular sieve (with FAU structure) with n (Si): n (Al) = 20.
1.2 mL of 3.2 g/L ruthenium chloride solution was added dropwise onto 1 g of the above molecular sieve. After drying at 80 °C for 2 hours, it was reduced in a fixed-bed reactor for 3 hours at 350 °C and a hydrogen flow rate of 10 mL/min to obtain the required catalyst.

2. Catalyst evaluation:

The catalyst evaluation method is shown in Example I-1. The composition and evaluation results of the catalyst are listed in Table I-15.

Comparative Example I-3

1. Catalyst preparation:

The catalyst was prepared with reference to Preparation Example I-1, except that the H-type silica-alumina molecular sieve was changed to an H-type MCM-22 molecular sieve (with MWW structure) with n (Si): n (Al) = 20.
1.2 mL of 3.2 g/L ruthenium chloride solution was added dropwise onto 1 g of the above molecular sieve. After drying at 80 °C for 2 hours, it was reduced in a fixed-bed reactor for 3 hours at 350 °C and a hydrogen flow rate of 10 mL/min to obtain the required catalyst.

2. Catalyst evaluation:

The catalyst evaluation method is shown in Example I-1. The composition and evaluation results of the catalyst are listed in Table I-15.

Comparative Example I-4

1. Catalyst preparation:

The catalyst was prepared with reference to Preparation Example I-1, except that the H-type silica-alumina molecular sieve was changed to an H-type MCM-22 molecular sieve (with MWW structure) with n (Si): n (Al) = 30.
1.2 mL of 3.2 g/L ruthenium chloride solution was added dropwise onto 1 g of the above molecular sieve. After drying at 80 °C for 2 hours, it was reduced in a fixed-bed reactor for 3 hours at 350 °C and a hydrogen flow rate of 10 mL/min to obtain the required catalyst.

2. Catalyst evaluation:

The catalyst evaluation method is shown in Example I-1. The composition and evaluation results of the catalyst are listed in Table I-15.

Table I-15 Catalyst composition and evaluation results of Comparative Examples I-1 to I-4

| Comparative Example | Types of molecular sieves | n(Si):n (Al) | Yield of cyclohexylbenzene (%) | Selectivity of cyclohexylbenzene (%) |
|---|---|---|---|---|
| I-1 | Y | 10 | 54 | 77.8 |
| I-2 | Y | 20 | 53 | 74.9 |
| I-3 | MCM-22 | 20 | 57 | 78.4 |
| I-4 | MCM-22 | 30 | 55 | 73.6 |

Example I-20

The catalyst prepared in Preparation Example I-1 was washed and dried before being put into the next reaction, and it was cycled for a total of 6 times. The reaction conditions in Example I-1 were retained for the catalyst evaluation, that is, 8 g of benzene was added to the high-pressure reactor, and hydrogen was charged to bring the system pressure to 1.2MPa. Then the temperature of the system was raised to 150 °C, and the reaction was completed after 4 hours. Catalyst evaluation results are listed in Table I-16.

Table I-16 Catalyst evaluation results of Example I-20

| Number of cycles | Yield of cyclohexylbenzene (%) | Selectivity of cyclohexylbenzene (%) |
|---|---|---|
| 1 | 59 | 93.7 |
| 2 | 59 | 93.2 |
| 3 | 60 | 93.4 |
| 4 | 59 | 93.8 |
| 5 | 59 | 92.9 |
| 6 | 59 | 93.5 |

Example I-21

The catalyst prepared in Preparation Example I-9 was washed and dried before being put into the next reaction, and it was cycled for a total of 6 times. The reaction conditions in Example I-12 were retained for the catalyst evaluation, that is, 8 g of benzene was added to the high-pressure reactor, and hydrogen was charged to bring the system pressure to 1.2MPa. Then the temperature of the system was raised to 150 °C, and the reaction was completed after 4 hours. Catalyst evaluation results are listed in Table I-17.

Table I-17 Catalyst evaluation results of Example I-21

| Number of cycles | Yield of cyclohexylbenzene (%) | Selectivity of cyclohexylbenzene (%) |
|---|---|---|
| 1 | 60 | 94.7 |
| 2 | 60 | 94.3 |
| 3 | 59 | 93.8 |
| 4 | 60 | 94.5 |
| 5 | 59 | 93.9 |
| 6 | 60 | 94.4 |

Preparation Example II-1

The Na-type MCM-22 molecular sieve (with MWW structure) with a silicon-aluminum molar ratio of 25:1 and 0.2mol/L $NH_4NO_3$ solution (mass ratio 1:20) were exchanged for ammonium ions at 45°C for 2 hours, and then centrifuged and washed. The sample obtained after repeating ammonium ion exchange twice was dried overnight at 100°C, and calcined in air at 550°C for 6 hours to obtain the H-type silica-alumina molecular sieve.

1.5 mL of 3.2 g/L ruthenium chloride solution (the solution concentration is based on ruthenium element, the same below) was added dropwise on 1 g of the H-type silica-alumina molecular sieve, and it was dried at 80°C for 2 hours to obtain a ruthenium-supported molecular sieve. The H$_2$-TPR test was conducted on the ruthenium-supported molecular sieve, and the reduction temperature was found to be 451°C.

0.2g of methyltrimethoxysilane, the ruthenium-supported molecular sieve and 10 mL of toluene solvent were mixed, refluxed at 110°C for 24h, centrifuged with water, washed, and dried at 80 °C for 12h to obtain the hydrocarbylated molecular sieve. The H$_2$-TPR test was conducted on the obtained hydrocarbylated molecular sieve, and the reduction temperature was found to be 476°C. This result shows that the active metal M supported on the hydrocarbylated molecular sieve is mainly distributed in the molecular sieve channels.

The hydrocarbylated molecular sieve was then reduced in a fixed-bed reactor for 3 hours at 450°C and a hydrogen volume space velocity of 50 h$^{-1}$ to obtain the target catalyst.

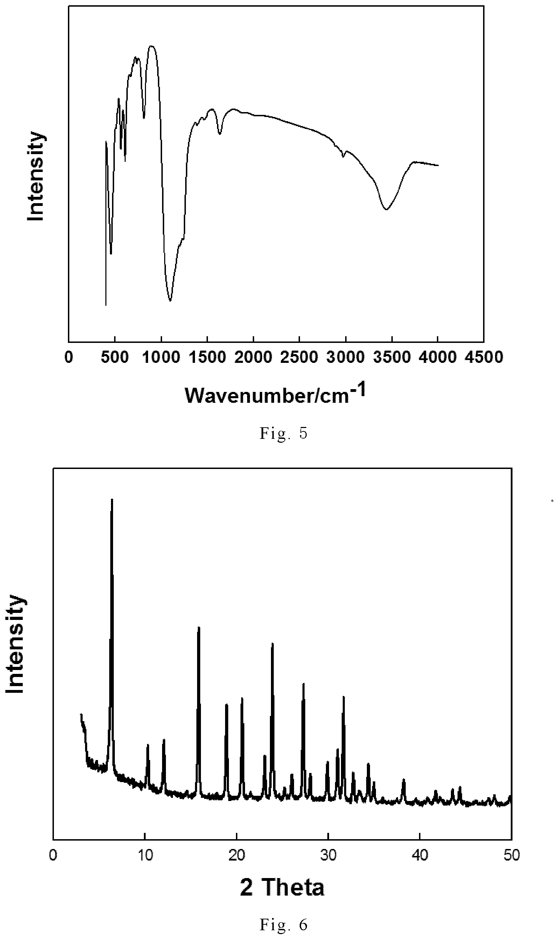

The XRD spectrum of the obtained catalyst is shown in Figure 4. According to XRD, it can be seen that the catalyst as a whole retains the MWW molecular sieve structure. The infrared absorption spectrum of the obtained catalyst is shown in Figure 5. It can be seen that a Si-C absorption peak appears near the wave number 2950 cm$^{-1}$. In addition, the hydrocarbyl is determined to be methyl through TG-MS test, and the content is as shown in Table II-1. The catalyst specific surface area, pore volume, acidic properties (including total acid content and external surface acid equivalent), metal M content and external surface metal M content are shown in Table II-1.

Preparation Example II-2

The Na-type MCM-22 molecular sieve (with MWW structure) with a silicon-aluminum molar ratio of 25:1 and 0.2mol/L NH$_4$NO$_3$ solution (mass ratio 1:20) were exchanged for ammonium ions at 45°C for 2 hours, and then centrifuged and washed. The sample obtained after repeating ammonium ion exchange twice was dried overnight at 100°C, and calcined in air at 550°C for 6 hours to obtain the H-type silica-alumina molecular sieve.

1.5 mL of 3.2 g/L ruthenium chloride solution was added dropwise onto 1 g of the H-type silica-alumina molecular sieve. After drying at 80°C for 2 hours, it was reduced in a fixed-bed reactor for 3 hours at 450°C and a hydrogen volume space velocity of 50h$^{-1}$ to obtain the catalyst precursor. 0.3g of dimethyldimethoxysilane, 1g of the catalyst precursor and 15mL toluene solvent were mixed, refluxed at 110°C for 24h, centrifuged with water, washed, and dried at 80 °C for 12h to obtain the catalyst.

The XRD spectrum of the obtained catalyst is the same as in Figure 1. The catalyst specific surface area, pore volume, acidic properties (including total acid content and external surface acid content), metal M content and external surface metal M content are shown in Table II-1.

Preparation Example II-3

The Na-type Y molecular sieve (with FAU structure) with a silicon-aluminum molar ratio of 10:1 and 0.2mol/L NH$_4$NO$_3$ solution (mass ratio 1:20) were exchanged for ammonium ions at 45°C for 2 hours, and then centrifuged and washed. The sample obtained after repeating ammonium ion exchange twice was dried overnight at 100°C, and calcined in air at 550°C for 6 hours to obtain the H-type silica-alumina molecular sieve.

2.5 mL of 3.2 g/L ruthenium chloride solution was added dropwise onto 1 g of the H-type silica-alumina molecular sieve. After drying at 80°C for 2 hours, it was reduced in a fixed-bed reactor for 3 hours at 450°C and a hydrogen volume space velocity of 100h$^{-1}$ to obtain the catalyst precursor. 0.3g of ethyltrimethoxysilane, 1g of the catalyst precursor and 15mL toluene solvent were mixed, refluxed at 110°C for 24h, centrifuged with water, washed, and dried at 80 °C for 12h to obtain the catalyst.

The XRD spectrum of the obtained catalyst is shown in Figure 6. The catalyst as a whole retains the FAU molecular sieve structure. The catalyst specific surface area, pore volume, acidic properties (including total acid content and external surface acid equivalent), metal M content and external surface metal M content are shown in Table II-1.

Preparation Example II-4

The Na-type Y molecular sieve (with FAU structure) with a silicon-aluminum molar ratio of 5:1 and 0.2mol/L NH$_4$NO$_3$ solution (mass ratio 1:20) were exchanged for ammonium ions at 45°C for 2 hours, and then centrifuged and washed. The sample obtained after repeating ammonium ion exchange twice was dried overnight at 100°C, and calcined in air at 550°C for 6 hours to obtain the H-type silica-alumina molecular sieve.

1.5 mL of 2.6 g/L palladium chloride solution (the solution concentration is based on palladium element, the same below) was added dropwise onto 1 g of the H-type silica-alumina molecular sieve. After drying at 80°C for 2 hours, it was reduced in a fixed-bed reactor for 3 hours at 450°C and a hydrogen volume space velocity of 100h$^{-1}$ to obtain the catalyst precursor. 0.2g of isopropyltrimethoxylsilane, 1g of the catalyst precursor and 10mL toluene solvent were mixed, refluxed at 110°C for 24h, centrifuged with water, washed, and dried at 80 °C for 12h to obtain the catalyst.

The XRD shows that the catalyst as a whole retains the FAU molecular sieve structure. The catalyst specific surface area, pore volume, acidic properties (including total acid content and external surface acid equivalent), metal M content and external surface metal M content are shown in Table II-1.

Preparation Example II-5

The Na-type Y molecular sieve (with FAU structure) with a silicon-aluminum molar ratio of 5:1 and 0.2mol/L $NH_4NO_3$ solution (mass ratio 1:20) were exchanged for ammonium ions at 45°C for 2 hours, and then centrifuged and washed. The sample obtained after repeating ammonium ion exchange twice was dried overnight at 100°C, and calcined in air at 550°C for 6 hours to obtain the H-type silica-alumina molecular sieve.

1.5 mL of nickel nitrate (6.0g/L)-copper nitrate (3.0g/L) mixed solution (the solution concentration is based on the metal elements, the same below) was added dropwise onto 1 g of the H-type silica-alumina molecular sieve. After drying at 80°C for 2 hours, it was reduced in a fixed-bed reactor for 3 hours at 450°C and a hydrogen volume space velocity of 100h$^{-1}$ to obtain the catalyst precursor. 0.2g of isopropyltrimethoxylsilane, 1g of the catalyst precursor and 10mL toluene solvent were mixed, refluxed at 110°C for 24h, centrifuged with water, washed, and dried at 80 °C for 12h to obtain the catalyst.

The XRD shows that the catalyst as a whole retains the FAU molecular sieve structure. The catalyst specific surface area, pore volume, acidic properties (including total acid content and external surface acid equivalent), metal M content and external surface metal M content are shown in Table II-1.

Preparation Example II-6

4g deionized water, 0.75g 4-pyrrolidinylpyridine, 0.42g aluminum isopropoxide, 1.5g silica sol, and 0.5g hydrofluoric acid were mixed uniformly to obtain a component with a composition of $1SiO_2:0.1Al_2O_3:1F^-:29H_2O:0.5OSDA$, followed by open heating pretreatment at 80 °C, and a block mixture was obtained. After the raw material mixture was heated and pretreated, the molar ratio of silicon source (calculated as $SiO_2$) and water during crystallization was 1:5, and then crystallized in a crystallizing kettle at 170 °C for 15 days. After taking out the product, it was washed three times with deionized water, dried and calcined at 550 °C for 6 hours to obtain the H-type ATS molecular sieve.

1.5 mL of 3.2 g/L ruthenium chloride solution was added dropwise onto 1 g of the H-type ATS molecular sieve. After drying at 80 °C for 2 hours, it was reduced in a fixed-bed reactor for 3 hours at 450°C and a hydrogen volume space velocity of 50h$^{-1}$ to obtain the catalyst precursor. 0.2g isopropyltrimethoxysilane, 1g catalyst precursor and 10mL toluene solvent were mixed, refluxed at 110°C for 24h, centrifuged with water, washed, and dried at 80°C for 12h to obtain the catalyst.

The XRD shows that the catalyst as a whole retains the ATS molecular sieve structure. The catalyst specific surface area, pore volume, acidic properties (including total acid content and external surface acid equivalent), metal M content and external surface metal M content are shown in Table II-1.

Preparation Example II-7

The catalyst was prepared with reference to Preparation Example II-1, except that the raw material was changed to the Na-type MCM-22 molecular sieve with a silicon-aluminum molar ratio of 50:1, and the remaining steps remained unchanged. The properties of the catalyst are shown in Table II-1.

The XRD spectrum of the obtained catalyst is similar to Figure 1. The catalyst specific surface area, pore volume, acidic properties (including total acid content and external surface acid content), metal M content and external surface metal M content are shown in Table II-1.

Examples II-1 to II-4

0.25g of the catalyst synthesized in Preparation Example II-1 was added to the high-pressure reactor, and then 8g of benzene was added to the reactor and hydrogen was charged. The reaction was completed after 4 hours. The specific evaluation conditions and evaluation data are shown in Table II-2.

Example II-5

0.25g of the catalyst synthesized in Preparation Example II-2 was added to the high-pressure reactor, and then 8g of benzene was added to the reactor and hydrogen was charged to bring the system pressure to 1.2 MPa. Then the temperature of the system was raised to 150 °C, and the reaction was completed after 4 hours. The evaluation data are summarized in Table 2.

Example II-6

0.25g of the catalyst synthesized in Preparation Example II-3 was added to the high-pressure reactor, and then 8g of benzene was added to the reactor and hydrogen was charged to bring the system pressure to 1.2 MPa. Then the temperature of the system was raised to 150 °C, and the reaction was completed after 4 hours. The evaluation data are summarized in Table II-2.

Example II-7

0.25g of the catalyst synthesized in Preparation Example II-4 was added to the high-pressure reactor, and then 8g of benzene was added to the reactor and hydrogen was charged to bring the system pressure to 1.2 MPa. Then the temperature of the system was raised to 150 °C, and the reaction was completed after 4 hours. The evaluation data are summarized in Table II-2.

Example II-8

0.25g of the catalyst synthesized in Preparation Example II-5 was added to the high-pressure reactor, and then 8g of benzene was added to the reactor and hydrogen was charged to bring the system pressure to 1.2 MPa. Then the temperature of the system was raised to 150 °C, and the reaction was completed after 4 hours. The evaluation data are summarized in Table II-2.

Example II-9

0.25g of the catalyst synthesized in Preparation Example II-6 was added to the high-pressure reactor, and then 8g of benzene was added to the reactor and hydrogen was charged to bring the system pressure to 1.2 MPa. Then the temperature of the system was raised to 150 °C, and the reaction was completed after 4 hours. The evaluation data are summarized in Table II-2.

Example II-10

0.25g of the catalyst synthesized in Preparation Example II-7 was added to the high-pressure reactor, and then 8g of benzene was added to the reactor and hydrogen was charged to bring the system pressure to 1.2 MPa. Then the temperature of the system was raised to 150 °C, and the reaction was completed after 4 hours. The evaluation data are summarized in Table II-2.

Comparative Example II-1

1. Catalyst preparation:

The catalyst was prepared with reference to Preparation Example II-1, except that the treatment step with methyltrimethoxysilane was omitted. The properties of the obtained catalyst are shown in Table II-1.

2. Catalyst evaluation:

The catalyst evaluation method is the same as in Example II-5, and the catalyst evaluation results are listed in Table II-2.

Comparative Example II-2

1. Catalyst preparation:

The catalyst was prepared with reference to Preparation Example II-1, except that the amount of the added ruthenium chloride solution of the same concentration was increased to 8 mL, that is, only the metal content in the catalyst was increased. The properties of the catalyst are shown in Table II-1.

2. Catalyst evaluation:

The catalyst evaluation method is the same as in Example II-5, and the catalyst evaluation results are listed in Table II-2.

Comparative Example II-3

1. Catalyst preparation:

The catalyst was prepared with reference to Preparation Example II-2, except that the addition of "0.3g dimethyldimethoxysilane" is increased to the addition of 1.0g dimethyldimethoxysilane, and the remaining steps remain unchanged. The properties of the catalyst are shown in Table II-1.

2. Catalyst evaluation:

The catalyst evaluation method is the same as in Example II-5, and the catalyst evaluation results are listed in Table II-2.

Comparative Example II-4

The catalyst obtained in Preparation Example II-1 was evaluated with reference to the method of Example II-5, in which the hydrogen partial pressure in the reaction conditions was changed to 4.0 MPa, and the other operations remained unchanged. The catalyst evaluation results are listed in Table II-2.

Table II-1 Physicochemical properties of the catalysts obtained in Preparation Examples and Comparative Examples

| Example number | Molecular sieve type | Metal M and content thereof (wt%) | Content of the metal M on the external surface to the external surface elements (wt%) | Distributi on coefficien t of the metal M on the external surface (%) | Hydrocarbyl modifying component and content thereof (wt%) | n (Si): n (Al) |
|---|---|---|---|---|---|---|
| Preparation Example II-1 | MCM-22 | 0.41 (ruthenium) | 0.23 | 16.4 | 2.4 (methyl) | 25 |
| Preparation Example II-2 | MCM-22 | 0.42 (ruthenium) | 0.19 | 13.3 | 5.3 (methyl) | 25 |
| Preparation Example II-3 | Y | 0.71 (ruthenium) | 0.34 | 9.9 | 3.9 (ethyl) | 10 |
| Preparation Example II-4 | Y | 0.41 (palladium) | 0.12 | 6.0 | 4.1 (isopropyl) | 5 |
| Preparation Example II-5 | Y | 0.38 (copper) 0.73 (nickel) | 0.11 0.28 | 6.7 | 4.1 (isopropyl) | 5 |
| Preparation Example II-6 | ATS type | 0.34 (ruthenium) | 0.11 | 1.7 | 4.2 (isopropyl) | 10 |
| Preparation Example II-7 | MCM-22 | 0.42 (ruthenium) | 0.22 | 13.4 | 2.4 (methyl) | 50 |

(continued)

| Example number | Molecular sieve type | Metal M and content thereof (wt%) | Content of the metal M on the external surface to the external surface elements (wt%) | Distributi on coefficien t of the metal M on the external surface (%) | Hydrocarbyl modifying component and content thereof (wt%) | n (Si): n (Al) |
|---|---|---|---|---|---|---|
| Comparativ e Example II-1 | MCM-22 | 0.41 (rutheniu m) | 0.39 | 29.5 | 0 | 25 |
| Comparativ e Example 11-2 | MCM-22 | 2.30 (rutheniu m) | 1.40 | 17.7 | 2.5 (methyl) | 25 |
| Comparative Example II-3 | MCM-22 | 0.40 (rutheniu m) | 0.04 | 2.9 | 22.3 (methyl) | 25 |

Table II-1 (continued) Physicochemical properties of the catalysts obtained in Preparation Examples and Comparative Examples

| Example number | External specific surface area (m$^2$/g) | Specific surface area (m$^2$/g) | Total pore volume (cm'/g) | Total acid content ($\mu$mol/g) | $C_{B, Py}$ / $C_{L, Py}$ | Relative acid equivalent of the external surface (%) |
|---|---|---|---|---|---|---|
| Preparation Example II-1 | 131 | 447 | 0.60 | 498 | 0.5 | 49 |
| Preparation Example II-2 | 136 | 464 | 0.61 | 477 | 0.5 | 38 |
| Preparation Example II-3 | 141 | 683 | 0.44 | 883 | 1.2 | 41 |
| Preparation Example II-4 | 138 | 677 | 0.44 | 878 | 1.2 | 41 |
| Preparation Example II-5 | 139 | 679 | 0.44 | 871 | 1.2 | 40 |
| Preparation Example II-6 | 23 | 434 | 0.27 | 1213 | 5.3 | 42 |
| Preparation Example II-7 | 118 | 461 | 0.61 | 439 | 0.5 | 44 |
| Comparative Example II-1 | 133 | 451 | 0.60 | 563 | 0.5 | 100 |
| Comparative Example II-2 | 128 | 439 | 0.58 | 493 | 0.5 | 50 |
| Comparative Example II-3 | 125 | 434 | 0.54 | 461 | 0.5 | 14 |

Table II-2 Catalytic evaluation conditions and results of Examples and Comparative Examples

| Example number | H$_2$ pressure (MPa) | Reaction temperature (°C ) | Yield of cyclohexylbenzene (%) | Selectivity of cyclohexylbenzene (%) |
|---|---|---|---|---|
| Example II-1 | 1.2 | 150 | 57 | 84.1 |
| Example II-2 | 1.6 | 150 | 61 | 83.6 |
| Example II-3 | 2.0 | 150 | 62 | 81.4 |
| Example II-4 | 1.2 | 180 | 55 | 84.6 |
| Example II-5 | 1.2 | 150 | 56 | 85.9 |
| Example II-6 | 1.2 | 150 | 57 | 84.7 |
| Example II-7 | 1.2 | 150 | 56 | 84.3 |

(continued)

| Example number | H$_2$ pressure (MPa) | Reaction temperature (°C ) | Yield of cyclohexylbenzene (%) | Selectivity of cyclohexylbenzene (%) |
|---|---|---|---|---|
| Example II-8 | 1.2 | 150 | 56 | 83.4 |
| Example II-9 | 1.2 | 150 | 58 | 93.7 |
| Example II-10 | 1.2 | 150 | 55 | 80.1 |
| Comparative Example II-1 | 1.2 | 150 | 53 | 77.8 |
| Comparative Example II-2 | 1.2 | 150 | 34 | 63.6 |
| Comparative Example II-3 | 1.2 | 150 | 27 | 79.8 |
| Comparative Example II-4 | 4.0 | 150 | 51 | 52.7 |

Example II-10

The catalyst prepared in Preparation Example II-1 was washed and dried before being put into the next reaction, and it was cycled for a total of 6 times, wherein the catalyst evaluation included that 8 g of benzene was added to the high-pressure reactor, and hydrogen was charged to bring the system pressure to 1.2MPa. Then the temperature of the system was raised to 150 °C, and the reaction was completed after 4 hours. Catalyst evaluation results are listed in Table II-3.

Table II-3 Catalyst evaluation results of Example II-10

| Number of cycles | Yield of cyclohexylbenzene (%) | Selectivity of cyclohexylbenzene (%) |
|---|---|---|
| 1 | 57 | 84.1 |
| 2 | 56 | 84.7 |
| 3 | 57 | 84.3 |
| 4 | 57 | 83.8 |
| 5 | 55 | 84.2 |
| 6 | 57 | 84.4 |

Preparation Example III-1

(1) Preparation of H-type MWW molecular sieve

The MCM-22 molecular sieve (with MWW structure) with a silicon-aluminum molar ratio of 20:1 and 0.2mol/L NH$_4$NO$_3$ solution (mass ratio 1:20) were exchanged for ammonium ions at 45°C for 2 hours, and then centrifuged and washed. The sample obtained after repeating ammonium ion exchange twice was dried overnight at 100°C, and calcined in air at 550°C for 6 hours to obtain the H-type silica-alumina molecular sieve.

(2) Preparation of catalyst

0.5 mL of 8 g/L ruthenium chloride solution was added dropwise onto 1 g of the H-type silica-alumina molecular sieve with a silicon-aluminum ratio of 20:1. After drying at 80°C for 2 hours, it was reduced in a fixed-bed reactor for 2 hours at 500°C and a hydrogen volume space velocity of 80h$^{-1}$ to obtain the catalyst precursor. 0.3g of phenyltrimethoxysilane, 1g of the catalyst precursor and 20mL ethanol solvent were mixed, refluxed at 75°C for 24h, centrifuged with water, washed, and dried at 80 °C for 12h to obtain the catalyst.

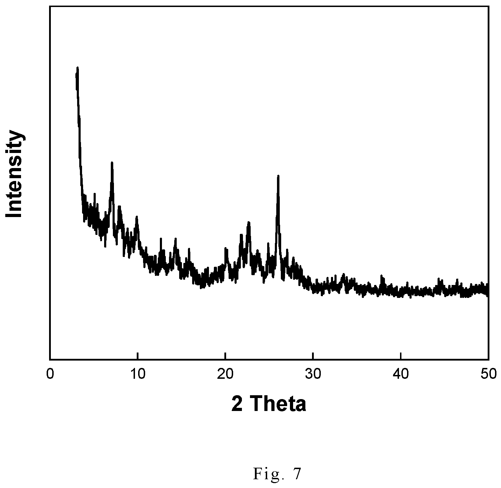

The XRD spectrum of the obtained catalyst is shown in Figure 7. The catalyst as a whole retains the MWW molecular sieve structure. The catalyst specific surface area, pore volume, acidic properties (including total acid content and external surface acid equivalent), metal M content and external surface metal M content are shown in Table III-1.

Preparation Example III-2

(1) Preparation of H-type MWW molecular sieve

The preparation process was the same as Preparation Example III-1, except that Na-type MCM-22 molecular sieve with a silicon-aluminum molar ratio of 25:1 was used.

(2) Preparation of catalyst

1 mL of 8 g/L ruthenium chloride solution was added dropwise onto 1 g of the H-type silica-alumina molecular sieve with a silicon-aluminum ratio of 25:1. After drying at 80°C for 2 hours, it was reduced in a fixed-bed reactor for 3 hours at 500°C and a hydrogen volume space velocity of $80h^{-1}$ to obtain the catalyst precursor. 0.2g of tolyltrimethoxysilane, 1g of the catalyst precursor and 20mL toluene solvent were mixed, refluxed at 110°C for 24h, centrifuged with water, washed, and dried at 80 °C for 12h to obtain the catalyst.

The XRD spectrum of the obtained catalyst is the same as Figure 7. The catalyst specific surface area, pore volume, acidic properties (including total acid content and external surface acid content), metal M content and external surface metal M content are shown in Table III-1.

Preparation Example III-3

(1) Preparation of H-type MOR molecular sieve

The preparation process was the same as Preparation Example III-1, except that Na-type mordenite (with MOR structure) with a silicon-aluminum molar ratio of 10:1 was used.

(2) Preparation of catalyst

0.5 mL of 8 g/L ruthenium chloride solution was added dropwise onto 1 g of the H-type MOR molecular sieve with a silicon-aluminum ratio of 10:1. After drying at 80°C for 2 hours, it was reduced in a fixed-bed reactor for 3 hours at 500°C and a hydrogen volume space velocity of $150h^{-1}$ to obtain the catalyst precursor. 0.3g of phenylsilanetriol, 1g of the catalyst precursor and 30mL toluene solvent were mixed, refluxed at 110°C for 24h, centrifuged with water, washed, and dried at 80 °C for 12h to obtain the catalyst.

The obtained catalyst as a whole retains the MOR molecular sieve structure. The catalyst specific surface area, pore volume, acidic properties (including total acid content and external surface acid equivalent), metal M content and external surface metal M content are shown in Table III-1.

Preparation Example III-4

(1) Preparation of H-type MOR molecular sieve

The preparation process was the same as Preparation Example III-1, except that Na-type mordenite (with MOR structure) with a silicon-aluminum molar ratio of 15:1 was used.

(2) Preparation of catalyst

0.5 mL of 8 g/L palladium chloride solution was added dropwise onto 1 g of the H-type MOR molecular sieve with a silicon-aluminum ratio of 15:1. After drying at 80°C for 2 hours, it was reduced in a fixed-bed reactor for 3 hours at 500°C and a hydrogen volume space velocity of $80h^{-1}$ to obtain the catalyst precursor. 0.2g of diphenylsilanediol, 1g of the catalyst precursor and 20mL ethanol solvent were mixed, refluxed at 75°C for 24h, centrifuged with water, washed, and dried at 80 °C for 12h to obtain the catalyst.

The obtained catalyst as a whole retains the MOR molecular sieve structure. The catalyst specific surface area, pore volume, acidic properties (including total acid content and external surface acid equivalent), metal M content and external surface metal M content are shown in Table III-1.

Preparation Example III-5

4g deionized water, 0.75g 4-pyrrolidinylpyridine, 0.42g aluminum isopropoxide, 1.5g silica sol, and 0.5g hydrofluoric acid were mixed uniformly to obtain a component with a composition of $1SiO_2:0.1Al_2O_3:1F^-:29H_2O:0.5OSDA$,

followed by open heating pretreatment at 80 °C, and a block mixture was obtained. After the raw material mixture was heated and pretreated, the molar ratio of silicon source (calculated as $SiO_2$) and water during crystallization was 1:5, and then crystallized in a crystallizing kettle at 170 °C for 15 days. After taking out the product, it was washed three times with deionized water, dried and calcined at 550 °C for 6 hours to obtain the H-type ATS molecular sieve.

0.5 mL of 8 g/L ruthenium chloride solution was added dropwise onto 1 g of the H-type ATS molecular sieve. After drying at 80 °C for 2 hours, it was reduced in a fixed-bed reactor for 3 hours at 500°C and a hydrogen volume space velocity of $80h^{-1}$ to obtain the catalyst precursor. 0.2g phenyltrimethoxysilane, 1g catalyst precursor and 10mL toluene solvent were mixed, refluxed at 110°C for 24h, centrifuged with water, washed, and dried at 80°C for 12h to obtain the catalyst.

The obtained catalyst as a whole retains the ATS molecular sieve structure. The catalyst specific surface area, pore volume, acidic properties (including total acid content and external surface acid equivalent), metal M content and external surface metal M content are shown in Table III-1.

Preparation Example III-6

The catalyst was prepared with reference to Preparation Example III-1, except that the raw material was changed to Na-type MCM-22 molecular sieve (with MWW structure) with a silicon-aluminum molar ratio of 50:1, and the remaining steps remained unchanged.

The XRD spectrum of the obtained catalyst was the same as Figure 7. The catalyst specific surface area, pore volume, acidic properties (including total acid content and external surface acid equivalent), metal M content and external surface metal M content are shown in Table III-1.

Examples III-1 to III-3

0.25g of the catalyst synthesized in Preparation Example III-1 was added to the high-pressure reactor, and then 10g of benzene was added to the reactor and hydrogen was charged. The reaction was completed after 4 hours. The specific evaluation conditions and evaluation data are shown in Table III-2.

Example III-4

0.25g of the catalyst synthesized in Preparation Example III-2 was added to the high-pressure reactor, and then 10g of benzene was added to the reactor and hydrogen was charged to bring the system pressure to 1.0 MPa. Then the temperature of the system was raised to 180 °C, and the reaction was completed after 4 hours. The evaluation data are summarized in Table III-2.

Example III-5

0.25g of the catalyst synthesized in Preparation Example III-3 was added to the high-pressure reactor, and then 10g of benzene was added to the reactor and hydrogen was charged to bring the system pressure to 1.0 MPa. Then the temperature of the system was raised to 180 °C, and the reaction was completed after 4 hours. The evaluation data are summarized in Table III-2.

Example III-6

0.25g of the catalyst synthesized in Preparation Example III-4 was added to the high-pressure reactor, and then 10g of benzene was added to the reactor and hydrogen was charged to bring the system pressure to 1.0 MPa. Then the temperature of the system was raised to 180 °C, and the reaction was completed after 4 hours. The evaluation data are summarized in Table III-2.

Example III-7

0.25g of the catalyst synthesized in Preparation Example III-5 was added to the high-pressure reactor, and then 10g of benzene was added to the reactor and hydrogen was charged to bring the system pressure to 1.0 MPa. Then the temperature of the system was raised to 180 °C, and the reaction was completed after 4 hours. The evaluation data are summarized in Table III-2.

Example III-8

0.25g of the catalyst synthesized in Preparation Example III-6 was added to the high-pressure reactor, and then 10g of benzene was added to the reactor and hydrogen was charged to bring the system pressure to 1.0 MPa. Then the temperature of the system was raised to 180 °C, and the reaction was completed after 4 hours. The evaluation data are summarized in Table III-2.

Comparative Example III-1

1. Catalyst preparation:

The catalyst was prepared with reference to Preparation Example III-1, except that the treatment with phenyltrimethoxysilane was omitted.

2. Catalyst evaluation:

The catalyst evaluation method is the same as in Example III-4, and the catalyst evaluation results are listed in Table III-2.

Comparative Example III-2

1. Catalyst preparation:

The catalyst was prepared with reference to Preparation Example III-1, except that the amount of the added ruthenium chloride solution of the same concentration was increased to 8 mL, that is, only the metal content in the catalyst was increased.

2. Catalyst evaluation:

The catalyst evaluation method is the same as in Example III-4, and the catalyst evaluation results are listed in Table III-2.

Comparative Example III-3

1. Catalyst preparation:

The catalyst was prepared with reference to Preparation Example III-1, except that the conditions of the arylation step were changed from adding "0.3g phenyltrimethoxysilane" to adding 0.8g phenyltrimethoxysilane.

2. Catalyst evaluation:

The catalyst evaluation method is the same as in Example III-4, and the catalyst evaluation results are listed in Table III-2.

Comparative Example III-4

The catalyst obtained in Preparation Example III-1 was evaluated according to the method of Example III-4, in which the hydrogen partial pressure in the reaction conditions was changed to 4.0 MPa, and the other operations remained unchanged. Catalyst evaluation results are listed in Table III-2.

Table III-1 Physicochemical properties of the catalysts obtained in Preparation Examples and Comparative Examples

| Example number | Molecular sieve type | Metal M and content thereof (wt%) | Content of the metal M on the external surface to the external surface elements (wt%) | Distribution coefficient of the metal M on the external surface (%) | Hydrocarbyl modifying component and content thereof (wt%) | n (Si): n (Al) |
|---|---|---|---|---|---|---|
| Preparation Example III-1 | MCM-22 | 0.26 (ruthenium) | 0.08 | 8.7 | 9.8 (phenyl) | 20 |
| Preparation Example III-2 | MCM-22 | 0.53 (ruthenium) | 0.17 | 9.2 | 6.7 (tolyl) | 25 |
| Preparation Example III-3 | Mordenite | 0.25 (ruthenium) | 0.08 | 2.5 | 11.7 (phenyl) | 10 |
| Preparation Example III-4 | Mordenite | 0.37 (palladium) | 0.07 | 1.5 | 15.8 (phenyl) | 15 |
| Preparation Example III-5 | ATS type | 0.27 (ruthenium) | 0.09 | 2.1 | 6.5 (phenyl) | 10 |
| Preparation Example III-6 | MCM-22 | 0.29 (ruthenium) | 0.08 | 7.3 | 9.8 (phenyl) | 50 |
| Comparative Example III-1 | MCM-22 | 0.27 (ruthenium) | 0.26 | 26.9 | 0 | 20 |
| Comparative Example III-2 | MCM-22 | 2.20 (ruthenium) | 1.30 | 17.2 | 9.8 (phenyl) | 20 |
| Comparative Example III-3 | MCM-22 | 0.28 (ruthenium) | 0.03 | 2.9 | 22.2 (phenyl) | 20 |

Table III-1 (continued) Physicochemical properties of the catalysts obtained in Preparation Examples and Comparative Examples

| Example number | Specific surface area (m$^2$/g) | External specific surface area (m$^2$/g) | Total pore volume (cm$^3$/g) | Total acid content (μmol/g) | Relative acid equivalent of the external surface (%) |
|---|---|---|---|---|---|
| Preparation Example III-1 | 441 | 124 | 0.59 | 507 | 26 |
| Preparation Example III-2 | 455 | 126 | 0.60 | 518 | 31 |
| Preparation Example III-3 | 432 | 34 | 0.33 | 1209 | 25 |
| Preparation Example III-4 | 441 | 36 | 0.31 | 1189 | 19 |
| Preparation Example III-5 | 434 | 27 | 0.27 | 1198 | 27 |
| Preparation Example III-6 | 461 | 122 | 0.60 | 438 | 29 |
| Comparative Example III-1 | 447 | 120 | 0.60 | 561 | 100 |
| Comparative Example III-2 | 435 | 127 | 0.60 | 501 | 28 |

(continued)

| Example number | Specific surface area (m2/g) | External specific surface area (m2/g) | Total pore volume (cm3/g) | Total acid content ($\mu$mol/g) | Relative acid equivalent of the external surface (%) |
|---|---|---|---|---|---|
| Comparative Example III-3 | 438 | 119 | 0.54 | 473 | 11 |

Table III-2 Catalyst evaluation conditions and results of Examples and Comparative Examples

| Example number | $H_2$ pressure (MPa) | Reaction temperature (°C ) | Yield of cyclohexylbenzene (%) | Selectivity of cyclohexylbenzene (%) |
|---|---|---|---|---|
| Example III-1 | 1.0 | 180 | 58 | 87.8 |
| Example III-2 | 1.5 | 180 | 60 | 86.6 |
| Example III-3 | 2.0 | 150 | 55 | 84.1 |
| Example III-4 | 1.0 | 180 | 57 | 83.3 |
| Example III-5 | 1.0 | 180 | 56 | 85.5 |
| Example III-6 | 1.0 | 180 | 55 | 83.4 |
| Example III-7 | 1.0 | 180 | 58 | 95.8 |
| Example III-8 | 1.0 | 180 | 56 | 86.1 |
| Comparative Example III-1 | 1.0 | 180 | 54 | 74.8 |
| Comparative Example III-2 | 1.0 | 180 | 38 | 66.6 |
| Comparative Example III-3 | 1.0 | 180 | 20 | 81.1 |
| Comparative Example III-4 | 4.0 | 180 | 53 | 54.9 |

Example III-9

The catalyst prepared in Preparation Example III-1 was washed and dried before being put into the next reaction, and it was cycled for a total of 6 times, wherein the catalyst evaluation included that 8 g of benzene was added to the high-pressure reactor, and hydrogen was charged to bring the system pressure to 1.2MPa. Then the temperature of the system was raised to 150 °C, and the reaction was completed after 4 hours.

Table III-3 Catalyst evaluation results of Example III-9

| Number of cycles | Yield of cyclohexylbenzene (%) | Selectivity of cyclohexylbenzene (%) |
|---|---|---|
| 1 | 58 | 87.8 |
| 2 | 57 | 87.2 |
| 3 | 57 | 87.0 |
| 4 | 58 | 87.7 |
| 5 | 56 | 87.1 |
| 6 | 58 | 87.0 |

Preparation Example IV-1

The Na-type Y molecular sieve (with FAU structure) with a silicon-aluminum molar ratio of 10:1 and 0.2mol/L $NH_4NO_3$ solution (mass ratio 1:20) were exchanged for ammonium ions at 45°C for 2 hours, and then centrifuged and washed. The sample obtained after repeating ammonium ion exchange twice was dried overnight at 100°C, and calcined in air at 550°C for 6 hours to obtain the H-type silica-alumina molecular sieve.

4.0 mL of 3.0 g/L palladium chloride solution was added dropwise onto 1 g of the H-type silica-alumina molecular sieve. After drying at 80°C for 2 hours, it was reduced in a fixed-bed reactor for 3 hours at 450°C and a hydrogen volume space velocity of $100h^{-1}$ to obtain the catalyst precursor. 0.2g of methyltrimethoxysilane, 1g of the catalyst precursor and 15mL toluene solvent were mixed, refluxed at 110°C for 24h, centrifuged with water, washed, and dried at 80 °C for 12h to obtain the catalyst.

The XRD spectrum of the obtained catalyst is same as Figure 7. The catalyst specific surface area, pore volume, acidic properties (including total acid content and external surface acid equivalent), metal M content and external surface metal M content are shown in Table IV-1.

Preparation Example IV-2

The Na-type Y molecular sieve (with FAU structure) with a silicon-aluminum molar ratio of 10:1 and 0.2mol/L $NH_4NO_3$ solution (mass ratio 1:20) were exchanged for ammonium ions at 45°C for 2 hours, and then centrifuged and washed. The sample obtained after repeating ammonium ion exchange twice was dried overnight at 100°C, and calcined in air at 550°C for 6 hours to obtain the H-type silica-alumina molecular sieve.

4.0 mL of 3.0 g/L palladium chloride solution was added dropwise onto 1 g of the H-type silica-alumina molecular sieve. After drying at 80°C for 2 hours, it was reduced in a fixed-bed reactor for 3 hours at 450°C and a hydrogen volume space velocity of $100h^{-1}$ to obtain the catalyst precursor. 0.3g of dimethyldimethoxysilane, 1g of the catalyst precursor and 15mL toluene solvent were mixed, refluxed at 110°C for 24h, centrifuged with water, washed, and dried at 80 °C for 12h to obtain the catalyst.

The XRD shows that the obtained catalyst as a whole retains the FAU molecular sieve structure. The catalyst specific surface area, pore volume, acidic properties (including total acid content and external surface acid equivalent), metal M content and external surface metal M content are shown in Table IV-1.

Preparation Example IV-3

The Na-type Y molecular sieve (with FAU structure) with a silicon-aluminum molar ratio of 10:1 and 0.2mol/L $NH_4NO_3$ solution (mass ratio 1:20) were exchanged for ammonium ions at 45°C for 2 hours, and then centrifuged and washed. The sample obtained after repeating ammonium ion exchange twice was dried overnight at 100°C, and calcined in air at 550°C for 6 hours to obtain the H-type silica-alumina molecular sieve.

4.0 mL of 2.5 g/L palladium chloride solution was added dropwise onto 1 g of the H-type silica-alumina molecular sieve. After drying at 80°C for 2 hours, it was reduced in a fixed-bed reactor for 3 hours at 450°C and a hydrogen volume space velocity of $100h^{-1}$ to obtain the catalyst precursor. 0.3g of dimethyldimethoxysilane, 1g of the catalyst precursor and 15mL toluene solvent were mixed, refluxed at 110°C for 24h, centrifuged with water, washed, and dried at 80 °C for 12h to obtain the catalyst.

The XRD shows that the obtained catalyst as a whole retains the FAU molecular sieve structure. The catalyst specific surface area, pore volume, acidic properties (including total acid content and external surface acid equivalent), metal M content and external surface metal M content are shown in Table IV-1.

Preparation Example IV-4

The Na-type Y molecular sieve (with FAU structure) with a silicon-aluminum molar ratio of 5:1 and 0.2mol/L $NH_4NO_3$ solution (mass ratio 1:20) were exchanged for ammonium ions at 45°C for 2 hours, and then centrifuged and washed. The sample obtained after repeating ammonium ion exchange twice was dried overnight at 100°C, and calcined in air at 550°C for 6 hours to obtain the H-type silica-alumina molecular sieve.

4.0 mL of 3.0 g/L palladium chloride solution was added dropwise onto 1 g of the H-type silica-alumina molecular sieve. After drying at 80°C for 2 hours, it was reduced in a fixed-bed reactor for 3 hours at 450°C and a hydrogen volume space velocity of $100h^{-1}$ to obtain the catalyst precursor. 0.3g of dimethyldimethoxysilane, 1g of the catalyst precursor and 15mL toluene solvent were mixed, refluxed at 110°C for 24h, centrifuged with water, washed, and dried at 80 °C for 12h to obtain the catalyst.

The XRD shows that the obtained catalyst as a whole retains the FAU molecular sieve structure. The catalyst specific surface area, pore volume, acidic properties (including total acid content and external surface acid equivalent), metal M content and external surface metal M content are shown in Table IV-1.

Preparation Example IV-5

The Na-type Y molecular sieve (with FAU structure) with a silicon-aluminum molar ratio of 10:1 and 0.2mol/L $NH_4NO_3$ solution (mass ratio 1:20) were exchanged for ammonium ions at 45°C for 2 hours, and then centrifuged and washed. The sample obtained after repeating ammonium ion exchange twice was dried overnight at 100°C, and calcined in air at 550°C for 6 hours to obtain the H-type silica-alumina molecular sieve.

4.0 mL of 3.0 g/L palladium chloride solution was added dropwise onto 1 g of the H-type silica-alumina molecular sieve. After drying at 80°C for 2 hours, it was reduced in a fixed-bed reactor for 3 hours at 450°C and a hydrogen volume space velocity of 100h⁻¹ to obtain the catalyst precursor. 0.3g of phenyltrimethoxysilane, 1g of the catalyst precursor and 15mL toluene solvent were mixed, refluxed at 110°C for 24h, centrifuged with water, washed, and dried at 80 °C for 12h to obtain the catalyst.

The XRD shows that the obtained catalyst as a whole retains the FAU molecular sieve structure. The catalyst specific surface area, pore volume, acidic properties (including total acid content and external surface acid equivalent), metal M content and external surface metal M content are shown in Table IV-1.

Example IV-1

0.25g of the catalyst synthesized in Preparation Example IV-1 was added to the high-pressure reactor, and then 10g of n-decane was added to the reactor and hydrogen was charged to bring the system pressure to 3.0 MPa. Then the temperature of the system was raised to 350 °C, and the reaction was completed after 3 hours. The evaluation data are summarized in Table IV-2.

Example IV-2

0.25g of the catalyst synthesized in Preparation Example IV-1 was added to the high-pressure reactor, and then 10g of n-decane was added to the reactor and hydrogen was charged to bring the system pressure to 3.5 MPa. Then the temperature of the system was raised to 350 °C, and the reaction was completed after 3 hours. The evaluation data are summarized in Table IV-2.

Example IV-3

0.25g of the catalyst synthesized in Preparation Example IV-1 was added to the high-pressure reactor, and then 10g of n-decane was added to the reactor and hydrogen was charged to bring the system pressure to 3.0 MPa. Then the temperature of the system was raised to 380 °C, and the reaction was completed after 3 hours. The evaluation data are summarized in Table IV-2.

Example IV-4

0.25g of the catalyst synthesized in Preparation Example IV-2 was added to the high-pressure reactor, and then 10g of n-decane was added to the reactor and hydrogen was charged to bring the system pressure to 3.0 MPa. Then the temperature of the system was raised to 350 °C, and the reaction was completed after 3 hours. The evaluation data are summarized in Table IV-2.

Example IV-5

0.25g of the catalyst synthesized in Preparation Example IV-3 was added to the high-pressure reactor, and then 10g of n-decane was added to the reactor and hydrogen was charged to bring the system pressure to 3.0 MPa. Then the temperature of the system was raised to 350 °C, and the reaction was completed after 3 hours. The evaluation data are summarized in Table IV-2.

Example IV-6

0.25g of the catalyst synthesized in Preparation Example IV-4 was added to the high-pressure reactor, and then 10g of n-decane was added to the reactor and hydrogen was charged to bring the system pressure to 3.0 MPa. Then the temperature of the system was raised to 350 °C, and the reaction was completed after 3 hours. The evaluation data are summarized in Table IV-2.

Example IV-7

0.25g of the catalyst synthesized in Preparation Example IV-5 was added to the high-pressure reactor, and then 10g of n-decane was added to the reactor and hydrogen was charged to bring the system pressure to 3.0 MPa. Then the temperature of the system was raised to 350 °C, and the reaction was completed after 3 hours. The evaluation data are summarized in Table IV-2.

Comparative Example IV-1

The Na-type Y molecular sieve (with FAU structure) with a silicon-aluminum molar ratio of 10:1 and 0.2mol/L $NH_4NO_3$ solution (mass ratio 1:20) were exchanged for ammonium ions at 45°C for 2 hours, and then centrifuged and washed. The sample obtained after repeating ammonium ion exchange twice was dried overnight at 100°C, and calcined in air at 550°C for 6 hours to obtain the H-type silica-alumina molecular sieve.

4.0 mL of 3.0 g/L palladium chloride solution was added dropwise onto 1 g of the H-type silica-alumina molecular sieve. After drying at 80°C for 2 hours, it was reduced in a fixed-bed reactor for 3 hours at 450°C and a hydrogen volume space velocity of 100h$^{-1}$ to obtain the required catalyst.

The catalyst specific surface area, pore volume, acidic properties (including total acid content and external surface acid equivalent), metal M content and external surface metal M content are shown in Table IV-1.

The catalyst evaluation method is shown in Example IV-1. The composition and evaluation results of the catalyst are listed in Table IV-2.

Comparative Example IV-2

The Na-type Y molecular sieve (with FAU structure) with a silicon-aluminum molar ratio of 10:1 and 0.2mol/L $NH_4NO_3$ solution (mass ratio 1:20) were exchanged for ammonium ions at 45°C for 2 hours, and then centrifuged and washed. The sample obtained after repeating ammonium ion exchange twice was dried overnight at 100°C, and calcined in air at 550°C for 6 hours to obtain the H-type silica-alumina molecular sieve.

4.0 mL of 3.0 g/L palladium chloride solution was added dropwise onto 1 g of the H-type silica-alumina molecular sieve. After drying at 80°C for 2 hours, it was reduced in a fixed-bed reactor for 3 hours at 450°C and a hydrogen volume space velocity of 100h$^{-1}$ to obtain the catalyst precursor. 0.8g of phenyltrimethoxysilane, 1g of the catalyst precursor and 15mL toluene solvent were mixed, refluxed at 110°C for 24h, centrifuged with water, washed, and dried at 80 °C for 12h to obtain the catalyst.

The catalyst specific surface area, pore volume, acidic properties (including total acid content and external surface acid equivalent), metal M content and external surface metal M content are shown in Table IV-1.

The catalyst evaluation method is shown in Example IV-1. The composition and evaluation results of the catalyst are listed in Table IV-2.

Table IV-1 Physicochemical properties of the catalysts obtained in Preparation Examples and Comparative Examples

| Example number | Molecular sieve type | Metal M and content thereof (wt%) | Content of the metal M on the external surface to the external surface elements (wt%) | Distribution coefficient of the metal M on the external surface (%) | Hydrocarbyl modifying component and content thereof (wt%) | n (Si): n (Al) |
|---|---|---|---|---|---|---|
| Preparation Example IV-1 | Y | 1.04 (palladium) | 0.47 | 9.1 | 2.3 (methyl) | 10 |
| Preparation Example IV-2 | Y | 1.02 (palladium) | 0.33 | 6.2 | 5.5 (methyl) | 10 |
| Preparation Example IV-3 | Y | 0.88 (platinum) | 0.33 | 6.2 | 5.5 (methyl) | 10 |
| Preparation Example IV-4 | Y | 1.03 (palladium) | 0.26 | 5.6 | 5.6 (methyl) | 5 |
| Preparation Example IV-5 | Y | 1.04 (palladium) | 0.26 | 4.4 | 9.6 (phenyl) | 10 |
| Comparative Example IV-1 | Y | 1.06 (palladium) | 1.22 | 24.1 | 0 | 10 |
| Comparative Example IV-2 | Y | 1.03 (palladium) | 0.16 | 2.7 | 21.8 (phenyl) | 10 |

Table IV-1 (continued) Physicochemical properties of the catalysts obtained in Preparation Examples and Comparative Examples

| Example number | External specific surface area $(m^2/g)$ | Specific surface area $(m^2/g)$ | Total pore volume $(cm^3/g)$ | Total acid content $(\mu mol/g)$ | $C_{B, Py} / C_{L, Py}$ | elative acid equivalent of the external surface (%) |
|---|---|---|---|---|---|---|
| Preparation Example IV-1 | 143 | 681 | 0.43 | 869 | 1.2 | 49 |
| Preparation Example IV-2 | 141 | 678 | 0.44 | 853 | 1.2 | 41 |
| Preparation Example IV-3 | 139 | 673 | 0.45 | 877 | 1.2 | 42 |
| Preparation Example IV-4 | 144 | 683 | 0.44 | 856 | 1.2 | 41 |
| Preparation Example IV-5 | 139 | 677 | 0.44 | 843 | 1.2 | 21 |
| Comparative Example IV-1 | 145 | 682 | 0.44 | 891 | 1.2 | 100 |
| Comparative Example IV-2 | 140 | 662 | 0.42 | 634 | 1.1 | 12 |

Table IV-2 Catalyst evaluation conditions and results of Examples and Comparative Examples

| Example number | $H_2$ pressure (MPa) | Reaction temperature (°C) | Conversion rate of n-decane (%) | Selectivity of isomerization product (%) |
|---|---|---|---|---|
| Example IV-1 | 3.0 | 350 | 44 | 85.1 |
| Example IV-2 | 3.5 | 350 | 46 | 81.2 |
| Example IV-3 | 3.0 | 380 | 45 | 86.8 |
| Example IV-4 | 3.0 | 350 | 47 | 87.3 |
| Example IV-5 | 3.0 | 350 | 48 | 84.8 |
| Example IV-6 | 3.0 | 350 | 46 | 87.1 |
| Example IV-7 | 3.0 | 350 | 48 | 89.3 |
| Comparative Example IV-1 | 3.0 | 350 | 36 | 83.2 |
| Comparative Example IV-2 | 3.0 | 350 | 37 | 90.6 |

The preferred embodiments of the present application have been described in detail above. However, the present application is not limited to the specific details in the above-mentioned embodiments. Within the scope of the technical concept of the present application, a variety of simple modifications can be made to the technical solutions of the present application. These simple modifications all fall within the protection scope of the present application.

In addition, it should be noted that each of the specific technical features described in the above-mentioned specific embodiments can be combined in any suitable manner without conflict. In order to avoid unnecessary repetition, the present application will not further explain the various possible combinations.

In addition, any combination can be made between various different embodiments of the present application, as long as they do not deviate from the idea of the present application, and they should also be regarded as the content invented by the present application.

**Claims**

1. A hydrogenation-acid catalysis bifunctional catalyst, based on the mass of the catalyst, containing 80-99.8% of a silica-alumina molecular sieve component, 0.2-2% of a metal component with hydrogenation activity supported on the molecular sieve, and 0-20% of a hydrocarbyl modifying component, wherein the hydrogenation active metal is selected from ruthenium, platinum, palladium, copper, nickel, or a combination thereof, more preferably selected from ruthenium, palladium, or a combination thereof, the hydrocarbyl modifying component is a $C_{1-20}$ hydrocarbyl, preferably a $C_{1-10}$ hydrocarbyl, more preferably selected from methyl, ethyl, propyl, isopropyl, butyl, phenyl, benzyl, phenethyl, or a combination thereof.

2. The catalyst according to claim 1, wherein the silica-alumina molecular sieve is selected from molecular sieves with MWW, FAU, MOR, BEA or ATS structure, or a combination thereof, preferably molecular sieves with ATS structure; preferably, the silicon-aluminum ratio of the silica-alumina molecular sieve is 2-50, preferably 2-40, and more preferably 2-20.

3. The catalyst according to claim 1 or 2, wherein the silica-alumina molecular sieve is a silica-alumina molecular sieve with ATS structure, and the X-ray diffraction spectrum of the catalyst shows the relative intensity characteristics of the diffraction peaks as shown in the following table:

| $2\theta(°)$ | Relative intensity $(I/I_0 \times 100)$ |
| --- | --- |
| 7.588-8.188 | vs |
| 16.286-16.886 | m-s |
| 18.927-19.527 | s |
| 20.492-21.092 | s |
| 22.096-22.696 | m-s |
| 26.983-27.583 | m-s |

4. The catalyst according to claim 3, wherein the X-ray diffraction spectrum of the catalyst shows the relative intensity characteristics of the diffraction peaks shown in any row of the following table:

| $2\theta(°)$ | Relative intensity $(I/I_0 \times 100)$ |
| --- | --- |
| 21.600-22.200 | m |
| 28.224-28.824 | w-m |
| 28.975-29.575 | w-m |
| 30.255-30.855 | w-m |
| 31.794-32.394 | m |
| 34.607-35.207 | m |

5. The catalyst according to any one of claims 1-4, wherein, based on the mass of the catalyst, the catalyst contains 80-98% of the silica-alumina molecular sieve component, 0.2-2% of the metal component with hydrogenation activity, and 1-20% of the hydrocarbyl modifying component, and according to X-ray photoelectron spectroscopy (XPS) test, the mass content of the hydrogenation active metal on the external surface of the catalyst to the elements on the external surface is 0.5% or less, preferably 0.4% or less;

preferably, based on the mass of the catalyst, the catalyst contains 90-98% of the silica-alumina molecular sieve component, 0.2-1.5% of the metal component with hydrogenation activity, and 1-10% of the hydrocarbyl modifying component;
further preferably, the distribution coefficient of the hydrogenation active metal on the external surface of the catalyst is 1-20%, preferably 1.5-18%.

6. The catalyst according to any one of claims 1-5, having one or more of the following characteristics:

the specific surface area of the catalyst is 200-800m$^2$/g, preferably 250-700m$^2$/g;
the total pore volume of the catalyst is not less than 0.15cm$^3$/g, preferably 0.18-1.0cm$^3$/g;

the micropore volume of the catalyst is 0.05-0.30 cm$^3$/g, preferably 0.10-0.25 cm$^3$/g;
the total acid content of the catalyst is 400-1500 $\mu$mol·g$^{-1}$, preferably 600-1500 $\mu$mol·g$^{-1}$;
the relative acid equivalent of the external surface of the catalyst is 15-50%, preferably 15-40%;
the metal H$_2$-TPR test reduction temperature of the catalyst is 470-500°C, preferably 480-500°C;
the acid content ratio of B acid/L acid of the catalyst is 0.2-8.0, preferably 0.4-6.0; and
in the catalyst, the crystals have a strip-like or rod-like morphology, the length of the crystals is 0.3-3 $\mu$m, and the aspect ratio is 2-20, preferably 5-20.

7. A method for preparing the catalyst according to any one of claims 1-6, comprising the following steps:

   (1) providing a H-type silica-alumina molecular sieve; and
   (2) supporting the hydrogenation active metal on the H-type silica-alumina molecular sieve, and optionally performing hydrocarbylation treatment and/or reduction on the resulting product to obtain the catalyst.

8. The method according to claim 7, wherein step (1) includes subjecting the silica-alumina molecular sieve raw material to ammonium ion exchange and calcining to obtain the H-type silica-alumina molecular sieve,
   preferably, the silica-alumina molecular sieve raw material is selected from silica-alumina molecular sieves with MWW, FAU, MOR or BEA structure, or a combination thereof.

9. The method according to claim 7, wherein step (1) includes mixing a silicon source, an aluminum source, a fluorine source, an organic structure directing agent and water, and after heating pretreatment, crystallization treatment and calcining are performed to obtain H-type ATS silica-alumina molecular sieve, wherein the silicon source is selected from silicic acid, silica gel, silica sol, tetraethyl silicate, sodium silicate, or a combination thereof, and the aluminum source is selected from pseudo-boehmite, aluminum isopropoxide, or a combination thereof, the fluorine source is hydrofluoric acid, and the organic structure directing agent is 4-pyrrolidinylpyridine;
   preferably, step (1) has one or more of the following characteristics:

   the molar ratio of the added silicon source, calculated as SiO$_2$, the aluminum source, calculated as Al$_2$O$_3$, the fluorine source, calculated as F$^-$, the organic structure directing agent and water is 1: (0.02-0.2): (0.5-2): (0.25-1.5): (3-15), preferably 1: (0.05-0.15): (0.5-1): (0.5-1): (5-10);
   in the crystallization treatment, the molar ratio of the silicon source, calculated as SiO$_2$, to water is 1: (1-10), preferably 1: (1.5-6.5); and
   the crystallization conditions include: the crystallization temperature is 120-200 °C, preferably 150-200 °C, and the crystallization time is 7-21 days, preferably 7-15 days.

10. The method according to any one of claims 7-9, wherein in step (2), the hydrogenation active metal is supported onto the H-type silica-alumina molecular sieve by adding a solution of a hydrogenation active metal source to the H-type silica-alumina molecular sieve and drying,
    preferably, step (2) has one or more of the following characteristics:

    the hydrogenation active metal source is selected from a soluble compound of the metal, preferably selected from a chloride, a nitrate of the metal, or a combination thereof;
    based on the mass of the hydrogenation active metal, the concentration of the solution of the hydrogenation active metal source is 1.5-50g/L, preferably 2-45g/L;
    the solution of the hydrogenation active metal source is added dropwise to the H-type silica-alumina molecular sieve; and
    the mass ratio of the hydrogenation active metal in the solution of the hydrogenation active metal source to the H-type silica-alumina molecular sieve is 0.002-0.015:1, for example 0.005-0.02:1.

11. The method according to any one of claims 7 to 10, wherein the hydrocarbylation treatment includes mixing and reacting the product supported with the hydrogenation active metal with a hydrocarbylation reagent in a solvent, wherein the hydrocarbylation reagent is selected from methyltrimethoxysilane, dimethyldimethoxysilane, ethyltrimethoxysilane, diethyldimethoxysilane, propyltrimethoxysilane, isopropyltrimethoxysilane, phenyltrimethoxysilane, tolyltrimethoxysilane, phenylsilanetriol, tolylsilanetriol, diphenylsilanediol, or a combination thereof, preferably selected from dimethyldimethoxysilane, diethyldimethoxysilane, isopropyltrimethoxysilane, phenyltrimethoxysilane, tolyltrimethoxysilane, phenylsilanetriol, tolylsilanetriol, or a combination thereof;
    preferably, the hydrocarbylation treatment includes one or more of the following characteristics:

the solvent is ethanol, toluene or a combination thereof;

the mass ratio of the product supported with the hydrogenated active metal, the hydrocarbylation reagent and the solvent is 1: (0.05-0.45): (5-55), preferably 1: (0.06-0.40): (6-50); and

the reaction conditions of the hydrocarbylation treatment include: the reaction temperature is 40-110 °C, preferably 70-110°C, and the reaction time is 6-48 hours, preferably 8-24 hours.

12. The method according to any one of claims 7 to 11, wherein the reduction is carried out using a reducing gas, preferably hydrogen, and the reduction conditions preferably include: the reduction temperature is 300-450 °C, the reduction time is 3-6 hours, and the volume space velocity of the reducing gas is $40\text{-}200h^{-1}$.

13. Use of the hydrogenation-acid catalysis bifunctional catalyst according to any one of claims 1 to 6 in hydrocarbon hydroconversion reaction, including contacting and reacting a hydrocarbon raw material with the catalyst in the presence of hydrogen,

preferably, the hydroconversion reaction is selected from benzene hydroalkylation reaction and alkane hydroisomerization reaction.

14. A one-step method for producing cyclohexylbenzene by hydrogenating benzene, including the step of contacting and reacting benzene with the hydrogenation-acid catalysis bifunctional catalyst according to any one of claims 1 to 6 in the presence of hydrogen to obtain cyclohexylbenzene;

preferably, the reaction conditions include: the mass ratio of benzene to the catalyst is 8-40, preferably 10-40; the reaction temperature is 100-220 °C, preferably 120-200 °C; the reaction time is 2-8 hours, preferably 2.5-6 hours; the hydrogen pressure is 0.8-2.5MPa, preferably 1.0-2.5MPa.

15. A method of alkane hydroisomerization, comprising contacting and reacting a linear alkane with the hydrogenation-acid catalysis bifunctional catalyst according to any one of claims 1 to 6 in the presence of hydrogen to obtain an isomerization product;

wherein, the linear alkane is a C8 or higher linear alkane, preferably a C8-C20 linear alkane, and more preferably a C8-C12 linear alkane;

preferably, the reaction conditions include: the mass ratio of the linear alkane to the catalyst is 10-100, preferably 10-50; the reaction temperature is 250-400°C, preferably 300-400°C; the reaction time is 3-10 hours, preferably 4-10 hours; the hydrogen pressure is 2.5-5.0MPa, preferably 3.0-4.0MPa.

Fig. 1

Fig. 2

Fig. 3

Fig. 4

Fig. 5

Fig. 6

Fig. 7

## INTERNATIONAL SEARCH REPORT

| International application No. |
|---|
| **PCT/CN2022/127278** |

### A. CLASSIFICATION OF SUBJECT MATTER

B01J 29/068(2006.01)i; C07C 13/28(2006.01)i

According to International Patent Classification (IPC) or to both national classification and IPC

### B. FIELDS SEARCHED

Minimum documentation searched (classification system followed by classification symbols)

B01J; C07C

Documentation searched other than minimum documentation to the extent that such documents are included in the fields searched

Electronic data base consulted during the international search (name of data base and, where practicable, search terms used)

CNABS, CNTXT, DWPI, SIPOABS, ENTXT, ENTXTC, Elsevier Science Direct, ISI Web of Science, 读秀, DUXIU, 超星科技数字图书馆, CHAOXING, 中国期刊网全文数据库, Chinese Journal Network Full-Text Database: 中国石油化工股份有限公司, CHB, 环己基苯, 苯基环己烷, cyclohexyl 1w benzene, phenylcyclohexane, 钌, 铂, 钯, 铜, 镍, Ruthenium, Platinum, Palladium, Copper, Nickel, "Ru", "Pt", "Pd", "Ni", "Cu", 加氢, 双功能, 催化剂, 分子筛, MWW, FAU, MOR, BEA, ATS, 烃基化, 烷基化, 有机硅, 硅烷化, 异构化

### C. DOCUMENTS CONSIDERED TO BE RELEVANT

| Category* | Citation of document, with indication, where appropriate, of the relevant passages | Relevant to claim No. |
|---|---|---|
| X | CN 109999893 A (ZHENGZHOU UNIVERSITY) 12 July 2019 (2019-07-12)<br>embodiment 1 | 1-4, 6-10, 12-15 |
| X | CN 105439802 A (CHINA PETROLEUM & CHEMICAL CORPORATION et al.) 30 March 2016 (2016-03-30)<br>description, paragraphs 6-14 | 1-6, 13-15 |
| Y | CN 105439802 A (CHINA PETROLEUM & CHEMICAL CORPORATION et al.) 30 March 2016 (2016-03-30)<br>description, paragraphs 6-14 | 7-12 |
| Y | CN 110563543 A (CHINA PETROLEUM & CHEMICAL CORPORATION et al.) 13 December 2019 (2019-12-13)<br>description, paragraphs 17 and 37-38, and embodiments 2-3 | 7-12 |
| X | CN 107930681 A (CHINA PETROLEUM & CHEMICAL CORPORATION et al.) 20 April 2018 (2018-04-20)<br>claims 1-10 | 1-4, 6-10, 12-15 |

☑ Further documents are listed in the continuation of Box C.    ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **02 December 2022** | **16 December 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

<div align="center">**INTERNATIONAL SEARCH REPORT**</div>

| | International application No. |
|---|---|
| | **PCT/CN2022/127278** |

### C.    DOCUMENTS CONSIDERED TO BE RELEVANT

| Category* | Citation of document, with indication, where appropriate, of the relevant passages | Relevant to claim No. |
|---|---|---|
| X | CN 107303512 A (SINOPEC YANGZI PETROCHEMICAL COMPANY LIMITED et al.) 31 October 2017 (2017-10-31)<br>    description, paragraphs [0005]-[0028] | 1-4, 6-10, 12-15 |
| X | CN 111085249 A (CHINA PETROLEUM & CHEMICAL CORPORATION et al.) 01 May 2020 (2020-05-01)<br>    claims 1-16 | 1-4, 6-10, 12-15 |
| X | CN 111085250 A (CHINA PETROLEUM & CHEMICAL CORPORATION et al.) 01 May 2020 (2020-05-01)<br>    claims 1-16 | 1-4, 6-10, 12-15 |
| X | CN 111250148 A (XIAMEN UNIVERSITY) 09 June 2020 (2020-06-09)<br>    claims 1-10 | 1-4, 6-10, 12-15 |
| X | US 4268699 A (PHILLIPS PETROLEUM CO.) 19 May 1981 (1981-05-19)<br>    embodiments | 1-4, 6-10, 12-15 |
| X | CN 106518602 A (CHINA PETROLEUM & CHEMICAL CORPORATION et al.) 22 March 2017 (2017-03-22)<br>    claims 1-10 | 1-6, 13-15 |
| X | CN 110563534 A (CHINA PETROLEUM & CHEMICAL CORPORATION et al.) 13 December 2019 (2019-12-13)<br>    description, paragraphs 6-18 | 1-6, 13-15 |

Form PCT/ISA/210 (second sheet) (January 2015)

**INTERNATIONAL SEARCH REPORT**
**Information on patent family members**

International application No.

**PCT/CN2022/127278**

| Patent document cited in search report | | | Publication date (day/month/year) | Patent family member(s) | | | Publication date (day/month/year) |
|---|---|---|---|---|---|---|---|
| CN | 109999893 | A | 12 July 2019 | None | | | |
| CN | 105439802 | A | 30 March 2016 | CN | 105439802 | B | 09 January 2018 |
| CN | 110563543 | A | 13 December 2019 | CN | 110563543 | B | 12 July 2022 |
| CN | 107930681 | A | 20 April 2018 | CN | 107930681 | B | 05 May 2020 |
| CN | 107303512 | A | 31 October 2017 | CN | 107303512 | B | 27 March 2020 |
| CN | 111085249 | A | 01 May 2020 | CN | 111085249 | B | 24 June 2022 |
| CN | 111085250 | A | 01 May 2020 | CN | 111085250 | B | 28 June 2022 |
| CN | 111250148 | A | 09 June 2020 | CN | 111250148 | B | 29 January 2021 |
| US | 4268699 | A | 19 May 1981 | None | | | |
| CN | 106518602 | A | 22 March 2017 | CN | 106518602 | B | 19 February 2019 |
| CN | 110563534 | A | 13 December 2019 | CN | 110563534 | B | 05 April 2022 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110015457 A1
- CN 104105679 A
- US 4219689 A
- CN 112934251 A
- US 5643440 A
- US 5302279 A
- US 6190532 B

**Non-patent literature cited in the description**

- *CHEMICAL ABSTRACTS,* 827-52-1
- *Molecular Catalysis,* 2017, vol. 442, 27-38